(19) Europäisches Patentamt
European Patent Office
Office européen des brevets

(11) EP 3 689 358 A1

(12) EUROPÄISCHE PATENTANMELDUNG

(43) Veröffentlichungstag:
**05.08.2020 Patentblatt 2020/32**

(51) Int Cl.:
***A61K 36/18*** (2006.01) ***B01F 13/00*** (2006.01)

(21) Anmeldenummer: **19154942.7**

(22) Anmeldetag: **31.01.2019**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Bionorica SE**
**92318 Neumarkt (DE)**

(72) Erfinder: **POPP, Michael**
**92318 Neumarkt (DE)**

(74) Vertreter: **Simandi, Claus**
**Badehausallee 55**
**27476 Cuxhaven (DE)**

(54) **VERFAHREN ZUR HERSTELLUNG VON PFLANZENMATERIALIEN MIT REDUZIERTER VARIANZ**

(57) Die Erfindung betrifft ein Verfahren zur Herstellung von standardisierten oder quantifizierten Pflanzenmaterialien mit reduzierter Varianz an Pflanzeninhaltsstoffen, insbesondere aus Arznei(mittel)pflanzen unter Einsatz eines Mischungsrechners.

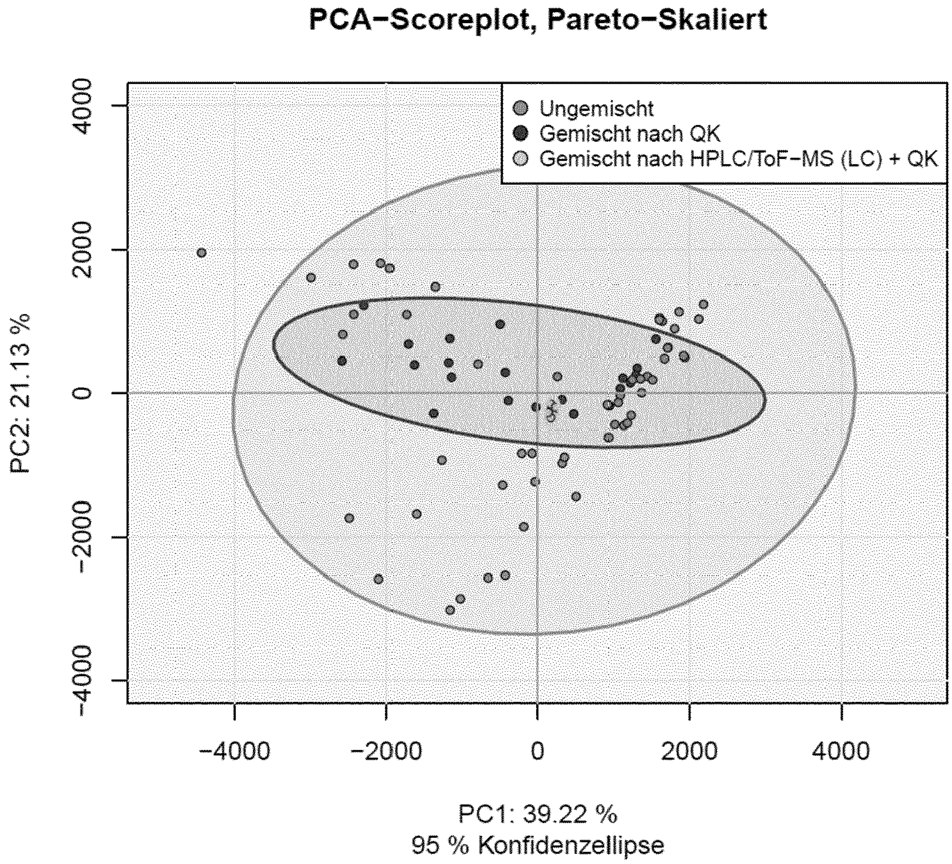


**Abbildung 4: Beispiel Score-Plot einer PCA der Mischungen von Primula veris**



Printed by Jouve, 75001 PARIS (FR)

**Beschreibung**

Die Erfindung betrifft ein Verfahren zur Herstellung von standardisierten oder quantifizierten Pflanzenmaterialien mit reduzierter Varianz an Pflanzeninhaltsstoffen, insbesondere aus Arznei(mittel)pflanzen unter Einsatz eines Mischungsrechners.

Arzneipflanzen enthalten, ggfs. in bestimmten Pflanzenteilen wie Wurzeln, Blättern, Blüten oder Früchten angereichert, Inhaltsstoffe mit pharmakologischer Wirkung und bilden die Grundlage für eine beachtliche Anzahl von Arzneimitteln. Zur Gewinnung dieser Inhaltsstoffe gibt es unterschiedliche Verfahren, von denen die meisten nach dem Prinzip einer wie auch immer gearteten Extraktion einschließlich Mazeration oder Perkolation der Pflanzen mit einem geeigneten Extraktionsmittel bzw. Lösungsmittel arbeiten und eine mehr oder weniger selektive Lösung und Anreicherung bestimmter pflanzlicher Wirkstoffe oder Wirkstoffgruppen in dem Extraktionsmittel bzw. Auszug ergeben. Die Extrakte können flüssig, halbfest oder fest und insbesondere ein Trockenextrakt (Extracta sicca) sein, wobei z.B. der Extraktionsrückstand, der erhaltene Fluidextrakt (Extracta fluida) bzw. die erhaltene Tinktur (Tincturae) zur Trockene eingeengt wird. Eine Trocknung kann mittels Wirbelschichttrocknung erfolgen oder eine Einengung zu einem Dick- oder Spissumextrakt (zähflüssiger Extrakt, Extracta spissa) erfolgen mit anschließender Vakuumbandtrocknung oder Hordentrocknung, siehe auch z.B. EP 0 753 306 B1 der Anmelderin.

Schaubild:

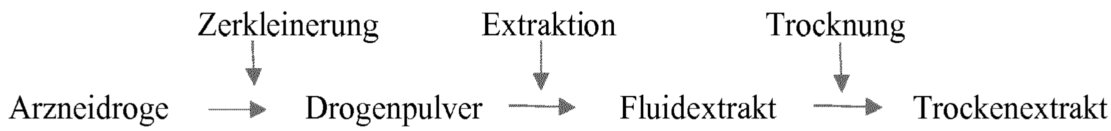


Die Anmelderin produziert und vertreibt z.B. solche Extrakte aus Arzneipflanzen, wie Bronchipret®, Sinupret Extract®, Canephron®, Imupret®, etc als auch auf Drogenbasis (Sinupret®).

Es handelt sich bei den in Rede stehenden Extrakten um registrierte oder zulassungspflichtige Arzneimittel, so genannte traditionelle oder rationale Phytopharmaka (inkl. well established medicinal use), die eine adäquate Dosierung als auch eine indikationsgerechte Verordnung unter Abwägung des Nutzen-Risiko-Verhältnisses bedürfen.

Monographien (auch: Arzneibuchvorschrift (Deutsches Arzneibuch (DAB), Europäisches Arzneibuch (EuAB oder Ph.Eur. für Pharmacopoea Europaea)) beschreiben Pflanzen zur Bereitstellung als Arzneimittel und deren Herstellung, insbesondere bezüglich ihrer wirksamkeitsbestimmenden Inhaltsstoffe, Wirkungen, Anwendungsgebiete, Gegenanzeigen, Nebenwirkungen, Wechselwirkungen, Dosierung und Darreichungsform. Positiv monographiert sind Pflanzen, für welche die Kommission E (Zulassungs- und Aufbereitungskommission am BfArM, Deutschland) oder das "Committee on Herbal Medicinal Products (HMPC)" am EMA einen ausreichenden Nachweis der Wirksamkeit und Unbedenklichkeit anhand der vorhandenen Studien aufführen konnte.

Gemäß Ph.Eur. und im Sinne dieser Erfindung sind Extrakte Zubereitungen von flüssiger, halbfester oder fester Beschaffenheit, die aus, üblicherweise getrockneten, pflanzlichen Drogen hergestellt werden.

So genannte *standardisierte Extrakte* werden innerhalb zulässiger Grenzen auf einem vorgegebenen Gehalt an wirksamkeitsbestimmenden Inhaltsstoffen (Leitsubstanzen) eingestellt. Die Einstellung kann durch Mischen von Extraktchargen erfolgen und / oder durch Zugabe von Hilfsstoffen.

So genannte *quantifizierte Extrakte* werden auf einen definierten Bereich von wirksamkeitsmitbestimmenden Inhaltsstoffen (Leitsubstanzen) eingestellt. Die Einstellung kann durch Mischen von Extraktchargen erfolgen.

Das Mischen von Extrakten bzw. Chargen kann durch eine Mischungsvorrichtung erfolgen, wobei ein Mischungsrechner eingesetzt wird, der das Mischverhältnis der Extrakte mithilfe eines computergestützten Rechners einstellt.

Pflanzenextrakte oder Drogenchargen bestehen jedoch aus einer Vielzahl von Pflanzeninhaltsstoffen. Der Gehalt von solchen Pflanzeninhaltsstoffen kann stark variieren, und zwar in Abhängigkeit von verschiedenen Parametern, wie zum Beispiel Anbauregion, Witterungsverhältnisse, Pflege, Erntemethoden, Erntezeitpunkt u.v.a.

Folglich enthalten Pflanzenextrakte oder Drogenchargen je nach Herkunft, Historie, Erntezeit, Herstellungsverfahren u.a. Parameter unterschiedliche Gehalte an Pflanzeninhaltsstoffen, welche zu qualitativen Unterschieden in den Einzelindividuen bzw. in einer Charge aus einer Gesamtheit von verarbeiteten Einzelindividuen als auch Chargen untereinander führen kann.

Daher weisen Pflanzenextrakte als auch pulverisierte Drogen das Problem der phytochemischen Chargenvariabilität auf. Um einen gleichbleibenden therapeutischen Erfolg bei einer Behandlung mit einem Phytopharmakon garantieren zu können, muss die Zusammensetzung des pflanzlichen Inhaltsstoffes eines Extrakts bzw. Phytopharmakons von Charge zu Charge möglichst gleich bzw. stabil bleiben, d.h. es besteht ein Bedürfnis an einer geringen Chargenvariabilität.

Dies ist letztlich darin begründet, dass die Pflanzeninhaltsstoffe der Extrakte oder Chargen eine natürliche, biologische Varianz ("biological space") aufweisen.

Pflanzeninhaltsstoffe sind insbesondere sekundäre Pflanzenstoffe. Sekundäre Pflanzenstoffe, bzw. Sekundärmetaboliten, leiten sich von Produkten des anabolen und katabolen Stoffwechsels ab, insbesondere von Kohlenhydraten und Aminosäuren. Für Arzneipflanzen sind die sekundären Pflanzenstoffe maßgeblich für die Eignung als Wirkstoff. Sekundäre Pflanzenstoffe umfassen insbesondere phenolische, isoprenoide und alkaloide Verbindungen, wie Phenole, Polyphenole, Flavonoide, Kaffeesäurederivate, Xanthone, Terpene, Steroide u.a. Naturstoffe. Insbesondere sekundäre Pflanzenstoffe können im Vorkommen und im Gehalt der Einzelindividuen bzw. Charge stark variieren.

Pflanzeninhaltsstoffe in Pflanzenmaterialien können durch analytische Methoden qualitativ und quantitativ charakterisiert werden (z.B. Chemotaxonomie). In erster Linie werden Gas- und/oder Flüssigchromatographie mit einem Massenspektrometer gekoppelt, wie GC-MS oder LC-MS. Üblich ist die Darstellung von Signalen (Peaks [m/z]) in Abhängigkeit von der Retentionszeit (Chromatogramm). Dies erlaubt ebenfalls die Bestimmung von Gehalten (z.B. w/w oder v/v) an Pflanzeninhaltsstoffen in der Droge oder im Extrakt bzw. in einem Pflanzenmaterial über erhaltene integrierte Peakflächen.

Im Stand der Technik sind keine Techniken beschrieben, die die Herstellung von Pflanzenextrakten oder Drogenchargen bzw. Pflanzenmaterialien erlaubt, wobei eine Reduktion der Varianz von Gehalten an Pflanzeninhaltsstoffen erreicht wird.

Daher ist es Aufgabe der vorliegenden Erfindung, Pflanzenmaterial(ien) bereitzustellen, die eine reduzierte Varianz (Variabilität) von Gehalten an Pflanzeninhaltsstoffen in einem Pflanzenmaterial aufweisen, insbesondere eine reduzierte Chargenvariabilität aufweisen.

Die Bereitstellung von Pflanzenmaterial(ien) mit reduzierter Varianz (synonym: Variabilität) von Gehalten an Pflanzeninhaltsstoffen führt zu einer optimierten Chargenhomogenität. Eine optimierte Charge erlaubt vorteilhaft eine verbesserte Reproduzierbarkeit der Chargen und folglich wird ein neuer Qualitätsstandard gewährleistet. Dies ist ebenfalls für Zulassungsfragen von hoher Bedeutung. Beispielsweise hat die FDA (US) bis heute keine Zulassung auf einen Pflanzenextrakt oder pulverisierte Drogen als Vielstoffgemisch gewährt. Weiterhin wird eine geringere Fehlertoleranz in vergleichenden Studien ermöglicht, welche wiederum die Studienqualität verbessert und mit synthetischen Arzneimitteln vergleichbar ist.

Daher betrifft die Erfindung zur Lösung dieser Aufgabe ein Verfahren zur Herstellung von Pflanzenmaterial mit reduzierter Varianz von Gehalten an Pflanzeninhaltsstoffen, wobei mindestens ein Varianzmarker verwendet wird und ein Mischen von mindestens zwei Chargen mittels eines Mischungsrechners erfolgt.

Die Aufgabe wird in einer bevorzugten Ausführungsform durch ein erfindungsgemäßes Verfahren zur Herstellung von Pflanzenmaterial mit reduzierter Varianz von Gehalten an Pflanzeninhaltsstoffen mit den folgenden Schritten gelöst (siehe ebenfalls **Beispiel B**):

i.) Bestimmung von Signalintensitäten zu Pflanzeninhaltsstoffen in zwei oder mehr Chargen mittels eines Detektors, insbesondere GC-MS und / oder LC-MS,
ii.) Identifizierung von mindestens einem Pflanzeninhaltsstoff, der einen Beitrag, vorzugsweise den größten Beitrag zur Varianz leistet und dessen Bestimmung der natürlichen Spannweite (nach- und vorstehend Varianzmarker genannt),
iii.) Festsetzen eines oder mehrerer Grenzwerte, welche kleiner als die jeweilige natürliche Spannweite aus ii.) ist,
iv.) Mischen von mindestens zwei Chargen, wobei mittels eines Mischungsrechners mindestens ein Grenzwert aus iii.) berücksichtigt wird,
v.) Optional, Wiederholung der Schritte i.) bis iv.).

Im Rahmen des Schrittes ii.) kann eine Bestimmung der Varianz der Gehalte von Pflanzeninhaltsstoffen durchgeführt werden.

Das erfindungsgemäße Verfahren erlaubt daher vorteilhaft die Herstellung von stabilen bzw. gleichen, homogenen Chargen, sodass insbesondere die Reproduzierbarkeit der Chargen gewährleistet ist. Dies ist insbesondere darin begründet, dass die Varianz der Pflanzeninhaltsstoffe reduziert ist und zwar über den identifizierten mindestens einen Varianzmarker, der eine maximale Reduktion der Gesamtvarianz der Pflanzeninhaltsstoffe erlaubt. Dieser erfindungsgemäß identifizierte Varianzmarker trägt maßgeblich zur Streuung bei und wird durch das erfindungsgemäße Verfahren in seiner Wirkung auf die Gesamtvariabilität reduziert.

Der Begriff "Gehalt an Pflanzeninhaltsstoffe" bedeutet die relative oder absolute Menge (Masse, Gewicht) eines oder mehrerer Pflanzeninhaltsstoffe(s) oder dessen/deren relative oder absolute Konzentration(en) (w/w) (v/v).

In einer weiteren bevorzugten Ausführungsform besteht das erfindungsgemäße Verfahren zur Herstellung von Pflanzenmaterial mit reduzierter Varianz von Gehalten an Pflanzeninhaltsstoffen mit den folgenden Schritten (siehe ebenfalls **Beispiel C**):

i.) Bestimmung von Signalintensitäten zu Pflanzeninhaltsstoffen in zwei oder mehr Chargen mittels eines Detektors, insbesondere GC-MS und / oder LC-MS,

ii.) Aufteilung der Signale in zwei oder mehrere Teilbereiche, sowie Aufsummierung der Signalintensitäten der Pflanzeninhaltsstoffe innerhalb eines jeden Teilbereichs,

iii.) Identifizierung von mindestens einem Teilbereich, der einen Beitrag, vorzugsweise den größten Beitrag zur Varianz leistet, und dessen Bestimmung der natürlichen Spannweite (nach- und vorstehend Varianzmarker genannt),

iv.) Festsetzen eines oder mehrerer Grenzwerte, welche kleiner als die jeweilige natürliche Spannweite aus iii.) ist,

v.) Mischen von mindestens zwei Chargen, wobei mittels eines Mischungsrechners mindestens ein Grenzwert aus iv.) berücksichtigt wird,

vi.) Optional, Wiederholung der Schritte i.) bis v.).

Im Rahmen des Schrittes iii.) kann eine Bestimmung der Varianz der Gehalte von Pflanzeninhaltsstoffen durchgeführt werden.

Diese Ausführungsform erlaubt besonders vorteilhaft die Bereitstellung von Pflanzenmaterialien aus komplexen Pflanzenmaterialien, die eine Vielzahl von Pflanzeninhaltsstoffen aufweisen, insbesondere mehr als 300 Pflanzeninhaltsstoffen, insbesondere von sekundären Pflanzenstoffen. Die erfindungsgemäßen Teilbereiche erlauben eine systematische Darstellung der komplexen Signalintensitäten unter Aufsummierung jener Signalintensitäten.

Allerdings können die Teilbereiche erfindungsgemäß weit als auch sehr eng gefasst sein (fokussiert), d.h. es kann z.B. jeder Teilbereich nur ein Signal eines Pflanzeninhaltsstoffs enthalten. Folglich kann diese Ausführungsform ebenfalls die erste Ausführungsform des Verfahrens (supra) umfassen.

Im Folgenden wird die Erfindung näher erläutert.

Der Gegenstand *"Bestimmung von Signalintensitäten zu Pflanzeninhaltsstoffen in zwei oder mehr Chargen mittels eines Detektors, insbesondere GC-MS und / oder LC-MS"* beinhaltet vorzugsweise den Einsatz von Flüssigchromatographie (LC), vorzugsweise HPLC, in Verbindung mit hochauflösender Massenspektrometrie, wie Time of Flight (TOF) Geräten, insbesondere die hochauflösende HPLC-TOF-MS. Die vorgenannten Begriffe GC-MS und / oder LC-MS sind nicht beschränkend zu verstehen, und umfassen jedwede Ausführungsform einer hierzu geeigneten Vorrichtung. Insbesondere können beliebige Detektoren eingesetzt werden, wie UV-VIS, Wärmeleitfähigkeitsdetektor (WLD), Flammenionisationsdetektor (FID) u.v.a. Es ist lediglich erforderlich, dass die eingesetzten Pflanzenmaterialien im Zuge der Bestimmung mittels eines Detektors, insbesondere GC-MS und / oder LC-MS entsprechende Signale (Peaks [m/z]) bzw. Signalintensitäten in Abhängigkeit der Retentionszeit darstellen können, sogenanntes Chromatogramm.

Daher sind solche Detektoren erfindungsgemäß ebenfalls umfasst, die Signalintensitäten in Abhängigkeit der Retentionszeit in einem Chromatogramm darstellen.

Sofern erforderlich, können die durch vorzugsweise GC-MS und / oder LC-MS identifizierten erfindungsgemäßen Varianzmarker bzw. entsprechend repräsentiert in den erfindungsgemäßen Teilbereichen durch weitere qualitätsbestimmende analytische Verfahren ergänzt werden (zum Beispiel: IR-, NIR-, Raman-Spektroskopie, Atomabsorbtionsspektroskopie, nasschemische Assays (z. B. Polyphenolbestimmung nach Folin-Ciocalteu), fragmentierende massenspektrometrische Techniken ($MS^n$)).

Bevorzugt ist, dass mindestens in einer Charge mindestens 100, 200 oder 300 Signale (bzw. Signalintensitäten) oder mehr bestimmt werden. Weiterhin ist bevorzugt, dass die Signalintensitäten in fünf Chargen, vorzugsweise verschieden voneinander, insbesondere zehn Chargen und mehr bestimmt werden.

In einer weiteren bevorzugten Ausführungsform, können die Signale bzw. Signalintensitäten zur jeweiligen Charge eines Pflanzenextrakts in einer Datenbank oder Speicher erfasst werden.

Der Gegenstand *"Bestimmung der Varianz der Gehalte von Pflanzeninhaltsstoffen"* kann wie folgt durchgeführt werden, wobei die bestimmten Signale bzw. Signalintensitäten zu einer Charge bzw. zu n Chargen in einer Matrix angeordnet werden:

$$\mathbf{A} = \begin{pmatrix} & Signal_1 & Signal_2 & \cdots & & Signal_k \\ Charge_1 & a_{1,1} & \cdots & \cdots & & a_{1,k} \\ Charge_2 & a_{2,1} & \ddots & \cdots & & a_{2,k} \\ \vdots & \vdots & \cdots & \ddots & \vdots & \\ Charge_n & a_{n,1} & \cdots & \cdots & & a_{n,k} \end{pmatrix}$$

Von jeder Spalte können die Mittelwerte $\overline{x_i}$ und die Standardabweichungen $s_i$ berechnet werden. Die mittlere relative Standardabweichung (in %) ergibt sich zu

$$RSDX = \frac{\sum_{i=1}^{k} s_i}{\sum_{i=1}^{k} \bar{x}_i}$$

Erfindungsgemäß kann die mittlere relative Standardabweichung aus der vorgenannten Matrix mathematisch bestimmt werden und folglich zur Bestimmung der Varianzreduktion herangezogen werden. Diese Bestimmung kann rechner-, computergestützt erfolgen.

Der Gegenstand *"Identifizierung von mindestens einem Pflanzeninhaltsstoff, der einen Beitrag, vorzugsweise den größten Beitrag zur Varianz leistet und dessen Bestimmung der natürlichen Spannweite (Varianzmarker)"* bzw. "*Identifizierung von mindestens einem Teilbereich, der einen Beitrag, vorzugsweise den größten Beitrag zur Varianz leistet, und dessen Bestimmung der natürlichen Spannweite (Varianzmarker)"* kann wie folgt durchgeführt werden:

Für die Ermittlung der Varianzmarker können alle mathematischen Verfahren verwendet werden, die aus einer Datenmatrix Variablen mit der größten Varianz identifizieren können. Dazu können z. B. in der einfachsten Form die Varianzen aller Variablen berechnet werden um anschließend die Variablen mit der größten Varianz auszuwählen. Bevorzugt sollte jedoch eine Principal component analysis (PCA, dt.: "Hauptkomponentenanalyse") zur Analyse herangezogen werden. Bei der PCA wird auf Basis der erhaltenen Daten ein sog. "Scoreplot" (Abbildung 1) erzeugt, bei dem die Gruppe der eingesetzten Chargen in einem Diagramm dargestellt werden. Je weiter die Punkte einer Gruppe (oder, wie in Abbildung 4 mehrerer Gruppen, berechnet innerhalb einer PCA) innerhalb des Score-Plots hierbei über das Koordinatensystem verteilt sind und je größer die sie umgebende Konfidenzellipse ist, desto größer ist die der Gruppe zugrundeliegende Varianz. Die PCA erzeugt zum anderen auch einen "Loadingplot" (siehe Abbildung 2), aus dem abgeleitet werden kann, welche Signale einen Beitrag, vorzugsweise den größten Beitrag zur Gesamtvarianz leisten und ggfs. zusätzlich mit anderen Signalen korrelieren. Die Auswahl der Varianzmarker wird in der Weise realisiert, dass der Loadingwert einer jeden Signalintensitätssumme der Teilbereiche über in diesem Fall 5 Hauptkomponenten (siehe ebenfalls **Beispiel D)**, Erklärung von > 80 % Gesamtvarianz) erst absolutiert, dann aufsummiert und schließlich die Loadingsummen absteigend sortiert werden. Um die gewählten Signale noch repräsentativer für den Gesamtextrakt zu machen, kann als Zusatzkriterium eine hohe Korrelation mit möglichst vielen anderen Signalen herangezogen werden. Die Loadingsummen mit dem höchsten resultierenden Werten enthalten jene Teilbereiche (und folglich Signal-Kandidaten), die als Varianzmarker verwendet werden können. Aus Abbildung 3 geht hervor, dass es zumeist eine begrenzte Anzahl von Teilbereichen (Signalen) gibt, die eine hohe Varianz aufzeigen.

Zweckmäßig ist es dann, für die erhaltenen Signale der gefundenen Varianzmarker den Gehalt absolut zu bestimmen (über LC-MS, LC-DAD, NIR oder andere analytische Verfahren) um vergleichbare Daten mehrerer Chargen, vorzugsweise gemessen über längere Zeiträume, zu erhalten.

Die erfindungsgemäß eingesetzte PCA ist im Sinne dieser Erfindung ein mathematisches Verfahren um aus einem sehr umfangreichen und komplexen Datensatz relevante Informationen zu extrahieren und das statistische Rauschen abzutrennen. Dies ist eine Technik des sog. "Data Minings", der einen Datensatz vereinfacht und dennoch einen möglichst großen Informationsgehalt beibehält.

Das Ergebnis einer PCA sind zumeist zwei Informationsblöcke: Ein sog. "Score-Plot" sowie ein hiermit verknüpfter "Loading-Plot". Im Score-Plot werden die Proben generell aufgrund ihrer "Eigenschaften" (erfindungsgemäß sind die Eigenschaften die Intensitätswerte der gemessenen (LC/MS-)Signale) gruppiert. Proben, die sich in all ihren Eigenschaften sehr ähnlich sind, sind eng zusammen gruppiert, solche, die sich stärker voneinander unterscheiden sind weiter voneinander entfernt. Die relative Ausdehnung der Punktwolke der Proben im Score-Plot (beispielsweise, wenn ungemischte mit gemischten Proben verglichen werden) sagt zudem etwas über ihre zugrundeliegende Variabilität aus. Eine kompakte Punktwolke hat eine geringere inhärente Varianz als eine ausgedehnte Punktwolke.

Der Loading-Plot dagegen zeigt an, welche Eigenschaften (oder hier: LC/MS-Signale) für die Positionierung der Objekte im Score-Plot maßgeblich verantwortlich sind. Signale, die im Loading-Plot weit vom Koordinatenursprung entfernt sind tragen stark zu einer Positionierung bei (und haben damit großen Einfluss auf die Variabilität der Proben) und sind daher ein vielversprechendes Optimierungskriterium im Rahmen der Mischungsberechnung.

Der Begriff *"Festsetzen eines oder mehrerer Grenzwerte welche kleiner als die jeweilige natürliche Spannweite ist"* bedeutet, dass Festsetzen eines Grenzwertes oder eines Bereiches von Gehalten welche kleiner als die natürliche Spannweite des mindestens einen Varianzmarkers ist. Der Wert 0 ist hierbei inbegriffen. Sind die Varianzmarker ermittelt, kann deren natürliche Spannweite aus den ermittelten Signalintensitäten bestimmt werden.

Der Gegenstand *"Mischen von mindestens zwei Chargen, wobei mittels eines Mischungsrechners mindestens ein festgesetzter Grenzwert berücksichtigt wird"* bedeutet, dass ein Algorithmus bereitgestellt wird, welcher mittels eines computergestützten Mischungsrechners berechnet, in welchem Verhältnis mindestens zwei oder mehr Chargen gemischt werden müssen, damit mindestens ein Varianzmarker innerhalb des neu gewählten, beschränkten Intervalls bzw. (Teil-)Bereiches liegt.

Für die Berechnung wird ein lineares Ungleichungssystem unter Berücksichtigung von Nebenbedingungen gelöst (z.B. Lay, David C. (August 22, 2005), Linear Algebra and Its Applications (3rd ed.), Addison Wesley). Wenn *n*

Chargen im Mischungspool zur Verfügung stehen und $k$ Grenzwertintervalle berücksichtigt werden, kann dieses als $\mathbf{G} * \vec{x} \geq \vec{h}$ geschrieben werden (FN 1). **G** ist hierbei eine Matrix mit $2 * n$ Zeilen und $k$ Spalten. Jede Zeile enthält die Werte der gemessenen Einzelparameter die optimiert werden sollen für jede Charge, wobei die Zeilen 1... $n$ ein positives Vorzeichen und die Zeilen $(n + 1)...2 * n$ ein negatives Vorzeichen erhalten. Der Vektor $\vec{h}$ enthält die Grenzen der Mischungsintervalle, wobei der Eintrag $h_1... h_{k/2}$ die unteren Grenzwerte und $h_{k/2+1}...h_k$ die oberen Grenzwerte enthält - auch diese müssen mit einem negativen Vorzeichen versehen werden. Bei der Lösung dieses Ungleichungssystem muss weiterhin eine Matrix mit Nebenbedingungen gleichzeitig mitberücksichtigt und gelöst werden. In dieser wird die Berechnung der prozentualen Anteile, sowie gegebenenfalls die Verwendung einzelner Chargen erzwungen. Sie wird als $\mathbf{A} * \vec{x} = \vec{b}$ formuliert, wobei **A** eine Matrix mit $m$ Zeilen (mit $m > 1$) und $k$ Spalten ist. Der Vektor $\vec{b}$ hat darüber hinaus ebenfalls $m$ Einträge, die stets den Wert 1 haben. Die erste Zeile von **A** hat ebenfalls nur 1 als Eintrag, in den weiteren Zeilen kann dagegen zusätzlich definiert werden, ob von einigen Chargen ein bewusst gewählter Mischungsanteil mitberücksichtigt werden soll, oder ob einzelne Chargen bewusst von der Berücksichtigung ausgeschlossen werden sollen.

Das derart formulierte Mischungsproblem wird mittels eines entsprechenden Algorithmus für lineare Optimierung gelöst und die zu mischenden Verhältnisse angegeben.

[1] Matrizen werden im Folgenden stets im Fettdruck geschrieben (z.B. **A**), Vektoren mit einem Pfeil (z.B. $\vec{x}$) und Skalare kursiv (z.B. $n$)).

In einer weiteren bevorzugten Ausführungsform wird die Varianz aus den Datensätzen vorzugsweise mittels einer Hauptkomponentenanalyse (Principal Component Analysis (PCA)) dargestellt. Insbesondere kann die PCA in einer weiteren bevorzugten Ausführungsform aus den Signalintensitätssummen der Teilbereiche eines Massendefekt-Plots erhalten werden. Bei einem Massendefekt-Plot werden die bestimmten Signale als $m/z$ gegen den Massendefekt aufgetragen. Der Massendefekt berechnet sich aus der Nachkommastellen der gemessenen Masse durch die Gesamtmasse der gemessenen Masse (siehe ebenfalls **Beispiel C**). Pflanzeninhaltsstoffe mit ähnlicher Masse und auch ähnlicher atomarer Zusammensetzung finden sich im Plot nahe beieinander (siehe Abbildung 11: Massendefektplot). Gemäß der bereits dargelegten bevorzugten Ausführungsform können die Teilbereiche nunmehr als Teilflächen ausgewiesen werden. Besonders vorteilhaft ist, dass im Zuge der Darstellung über den Massendefekt-Plot solche Teilflächen sekundäre Pflanzenstoffe repräsentieren können wie Phenole, Flavonoide u.v.a. und die Identifizierung eines Varianzmarkers für diesen Teilbereich bzw. Teilfläche leicht erfolgen kann.

Im Rahmen dieser Erfindung wird unter einer Charge, insbesondere Pflanzenmaterialiencharge, wie Drogen- oder Extraktcharge, die Gesamtheit der Einheiten verstanden, die in einem Chargenprozess produziert werden, die zur Herstellung von abgegrenzten Stoffmengen führt, indem Mengen von Einsatzstoffen unter Nutzung einer oder mehrerer Einrichtungen innerhalb eines abgegrenzten Zeitraums einer geordneten Folge von Prozessaktivitäten unterzogen werden. Ausgangsprodukt ist zumeist die Pflanzendroge aus welcher der Pflanzenextrakt oder die aufbereitete Pflanzendroge erhalten wird.

Das Mischen von zwei oder mehreren Chargen kann erfindungsgemäß in einem Mischer erfolgen, wobei die Zuteilung der einzelnen Chargen in eine Mischung durch den Mischungsrechner vorgegeben ist, welcher wiederum durch einen Algorithmus programmiert werden kann, der insbesondere den erfindungsgemäßen Grenzwert für einen Varianzmarker gemäß dem erfindungsgemäßen Verfahren berücksichtigt.

Pflanzenmaterialien (Einzahl oder Plural) im Sinne dieser Erfindung umfasst jedwedes pflanzliche Material, wie Pflanzenteile, solche wie Blätter, Stängel, Wurzel, Blüten. Insbesondere können Pflanzenmaterialien in Form ihrer Pflanzendrogen als auch Pflanzenextrakte (supra) vorliegen.

Weiterhin umfasst die Erfindung die erhaltenen Pflanzenmaterialien, insbesondere Pflanzenextrakte, die durch das erfindungsgemäße Verfahren erhalten werden können. Die erfindungsgemäßen erhaltenen Pflanzenmaterialien, insbesondere Pflanzenextrakte, weisen zumindest geänderte Gehalte von Pflanzeninhaltsstoffen auf, insbesondere weisen diese erhaltenen Pflanzenmaterialien, insbesondere Pflanzenextrakte, gegenüber dem Ausgangspflanzenmaterial eine reduzierte Varianz von Gehalten an Pflanzeninhaltsstoffen auf. Die erhaltenen Pflanzenmaterialien, insbesondere Pflanzenextrakte, sind hinsichtlich der reduzierten Varianz spezifisch, wobei mindestens ein Varianzmarker einen geänderten Gehalt an Pflanzeninhaltsstoff aufweist. Zudem ist die Streuung der erhaltenen Gehalte an Pflanzeninhaltsstoffen reduziert.

Daher betrifft die Erfindung ein Pflanzenmaterial mit reduzierter Varianz von Gehalten an Pflanzeninhaltsstoffen, welche gemäß den erfindungsgemäßen Verfahren erhalten oder hergestellt wird bzw. erhältlich ist.

Im Rahmen dieser Erfindung wird unter einem "Pflanzenextrakt" als auch "Pflanzenmaterial" ein Vielkomponentengemisch aus Naturstoffen verstanden, welches mehr als zwei Naturstoffe, insbesondere mehr als 10 oder 100 Naturstoffe, insbesondere mehr als 200, 300, 500 oder 1.000 Naturstoffe enthält. Pflanzenextrakte können beispielsweise mittels Extraktion, Perkolation oder Mazeration aus Pflanzenmaterialien gewonnen werden. Als Extraktionsmittel können Lösungsmittel, wie Wasser, C1-C5-Alkohole, Ethanol, oder andere Lösungsmittel mit ausreichender Polarität eingesetzt werden. Eine übliche Extraktion ist beispielsweise ein Gemisch aus Wasser / Ethanol (50:50, 70:30, 30:70).

Nachstehende Gattungen, insbesondere solche Arznei(mittel)pflanzen sind für die Pflanzenextrakte als auch

Pflanzenmaterialien erfindungsgemäß bevorzugt:
Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena, Sambucus, Gentiana, Cannabis, Silybum.

Nachstehende Arten, insbesondere solche Arznei(mittel)pflanzen sind für die Pflanzenextrakte als auch Pflanzenmaterialien erfindungsgemäß bevorzugt:
Equiseti herba (Schachtelhalmkraut), Juglandis folium (Walnussblätter), Millefolii herba (Schafgarbenkraut), Quercus cortex (Eichenrinde), Taraxaci herba (Löwenzahnkraut), Althaeae radix (Eibischwurzel) und Matricariae flos (bzw. Flos chamomillae (Kamilleblüten)), Centaurium erythraea (Tausendgüldenkraut), Levisticum officinale (Liebstöckel), Rosmarinus officinalis (Rosmarin), Angelica dahurica (Sibirische Engelwurz, PinYin Name: Baizhi), Angelica sinensis (Chinesische Angelika, PinYin Name: Danggui), Artemisia scoparia (Besen-Beifuß, PinYin Name: Yinchen), Astragalus membranaceus (var. Mongolicus) (Traganthwurzel, Chin.: Huang-Qi), Leonurus japonicus (Löwenohr, Chin.: T'uei), Salvia miltiorrhiza (Rotwurzel-Salbei, Chin.: Danshen), Saposhnikovia divaricata (Siler, PinYin Name: Fangfeng), Scutellaria baicalensis (Baikal-Helmkraut, Banzhilian), Siegesbeckia pubescens (Himmlisches Kraut, PinYin Name: Xixianciao), Armoracia rusticana (Meerrettich), Capsicum sp.(Paprika), Cistus incanus (Zistrose), Echinacea angustifolia (Sonnenhut), Echinacea purpurea (Sonnenhut), Galphimia glauca, Hedera helix (Efeu), Melia toosendan (Chinesische Holunderfrüchte, Chin.: Chuan Lian Zi), Olea europaea (Olive), Pelargonium sp. (Pelagornien), Phytolacca americana (Kermesbeeren), Primula veris (Schlüsselblume), Salix sp. (Weide), Thymus L. (Thymian), Vitex agnus castus (Mönchspfeffer), Vitis vinifera (Edle Weinrebe), Rumicis herba (Sorrel herb), Verbena officinalis (Eisenkraut), Sambucus nigra (Schwarzer Holunder), Gentiana lutea (Gelber Enzian), Cannabis sativa (Hanf), Silybum marianum (Mariendistel).

Ebenfalls sind Mischungen der vorstehenden Gattungen und / oder Arten erfindungsgemäß umfasst.

Beispiele und Abbildungen:

Nachfolgende Beispiele dienen zur näheren Erläuterung der Erfindung, ohne jedoch die Erfindung auf diese Beispiele einzuschränken.

Die Abbildungen 1-12 sind vor- und nachstehend erläutert.

Abbildung 13 fasst die in Beispiel B, C und D aufgeführten Möglichkeiten zur Bestimmung der Varianzmarker in einer Übersicht zusammen. Diese Ausführungsformen sind nicht abschließend zu lesen.

**Beispiel A**

**Beispiele für den Mischungserfolg**

Primula veris

Für die Droge Primula veris wurden zunächst mittels HPLC/ToF-MS 30 ungemischte Chargen vermessen, deren natürliche Varianz (d.h. der RSDX (supra)) bestimmt und anschließend nach dem oben beschriebenen Verfahren die Varianzmarker ermittelt. Mit dieser Information wurde sodann eine Mischungsberechnung für mehrere Ansätze durchgeführt, diese im Labor konkret hergestellt und erneut mittels HPLC/ToF-MS vermessen. Für die Mischungsberechnung und nachfolgende Vermessung wurden 5 Varianzmarker mit den höchsten absoluten summierten Loadingwerten verwendet. Eine Gegenüberstellung der so erzielbaren Werte für den RSDX ist in Abbildung 5 dargestellt. Für das Beispiel von Primula veris wurde außerdem eine PCA mit Daten gemischter und ungemischter Proben berechnet und um jede Probengruppe eine Konfidenzellipse gelegt (Abbildung 4). Je geringer der Flächeninhalt dieser Ellipse, desto geringer die Varianz, die hier wie erwartet in den gemischten Proben am geringsten ist. Dies visualisiert den gleichen Sachverhalt auf eine alternative Art und Weise zum RSDX (supra).

Rumex crispus

Für die Droge *Rumex crispus* wurden ebenfalls zunächst 40 ungemischte Chargen vermessen und dann wie für Primula veris verfahren. Eine Gegenüberstellung der erzielten Werte für den RSDX ist in der Abbildung 6 dargestellt.

Sambuccus nigra

Für die Droge *Sambuccus nigra* wurden ebenfalls zunächst 40 ungemischte Chargen vermessen und dann wie für *Primula veris* verfahren. Eine Gegenüberstellung der erzielten Werte für den RSDX ist in der Abbildung 7 dargestellt.

Verbena officinalis

Für die Droge Verbena officinalis wurden ebenfalls zunächst 30 ungemischte Chargen vermessen und dann wie für Primula veris verfahren. Eine Gegenüberstellung der erzielten Werte für den RSDX ist in der Abbildung 8 dargestellt.

**Beispiel B**

**Beispiel für das Vorgehen bei der Varianzreduktion von Pflanzenmaterialien durch Mischungsrechnung bei Rumex crispus**

Es werden Ausschnitte der originären Datensätze gezeigt, die das erfindungsgemäße Vorgehen hinreichend vermitteln.

i) Bestimmung von Signalen von Pflanzeninhaltsstoffen mittels LC/MS an mehreren Chargen

Das Ergebnis ist typischerweise eine Tabelle wie die Folgende - die Zahlen sind die gemessenen Intensitätswerte beispielsweise aus einer LC/MS-Messung.

| | Signal 1 | Signal 2 | Signal 3 | Signal 4 | Signal 5 | Signal 6 | Signal 7 | Signal 8 | Signal 9 | Signal 10 | Signal 11 | Signal 12 |
|---|---|---|---|---|---|---|---|---|---|---|---|---|
| Charge_1 | 16711 | 5763 | 14366 | 8912 | 8521 | 24000 | 29252 | 11208 | 9471 | 8302 | 7189 | 5317 |
| Charge_2 | 16797 | 5961 | 14480 | 8713 | 9170 | 23294 | 25059 | 5834 | 7969 | 8250 | 5102 | 3937 |
| Charge_3 | 16411 | 6182 | 14636 | 8096 | 7357 | 23788 | 21161 | 7111 | 9148 | 7458 | 4075 | 5450 |
| Charge_4 | 16800 | 7030 | 12939 | 8800 | 8293 | 24433 | 19279 | 6319 | 9599 | 8005 | 4291 | 6829 |
| Charge_5 | 16677 | 6739 | 13440 | 9141 | 9954 | 23389 | 20415 | 6159 | 10361 | 8324 | 2171 | 6645 |

ii) Bestimmung der Gesamtvarianz / -standardabweichung der 5 Samples

| | Signal 1 | Signal 2 | Signal 3 | Signal 4 | Signal 5 | Signal 6 | Signal 7 | Signal 8 | Signal 9 | Signal 10 | Signal 11 | Signal 12 |
|---|---|---|---|---|---|---|---|---|---|---|---|---|
| Mittelwert | 16679 | 6335 | 13972 | 8733 | 8659 | 23781 | 23033 | 7326 | 9310 | 8068 | 4566 | 5636 |
| Standardabweichung | 159 | 533 | 742 | 390 | 973 | 465 | 4100 | 2220 | 872 | 364 | 1818 | 1169 |
| rel. Standard-abweichung (in %) | 0,95 | 8,41 | 5,31 | 4,47 | 11,23 | 1,95 | 17,80 | 30,31 | 9,36 | 4,51 | 39,81 | 20,74 |

| | |
|---|---|
| Summe der Mittelwerte | 136097 |
| Summe der Standardabweichungen | 13804 |
| mittlere relative Standardabweichung (RSDX) in % | 10,14 |

iii) Identifizierung der Signale, die am stärksten zur Varianz / Standardabweichung beitragen

Berechnung der Loadings der Tabelle aus Schritt i) mittels einer Principal component analysis (PCA) (z.B. 5 Hauptkomponenten):

| | PC1 | PC2 | PC3 | PC4 | PC5 |
|---|---|---|---|---|---|
| Signal1 | 0,109279 | 0,412709 | -0,046291 | -0,523313 | -0,689587 |
| Signal2 | 0,429376 | -0,111223 | -0,019776 | -0,179957 | 0,030141 |
| Signal3 | -0,416814 | -0,070444 | 0,164048 | 0,221163 | -0,299226 |
| Signal4 | 0,214981 | 0,454797 | -0,077392 | 0,11073 | 0,361717 |
| Signal5 | 0,182568 | 0,418618 | 0,271278 | 0,18247 | 0,151153 |
| Signal6 | 0,090994 | -0,153925 | -0,571312 | -0,340579 | 0,308789 |
| Signal7 | -0,350032 | 0,291495 | -0,176306 | 0,116159 | 0,05639 |
| Signal8 | -0,231543 | 0,129218 | -0,49517 | 0,318806 | -0,107474 |
| Signal9 | 0,313837 | -0,004629 | -0,276986 | 0,533461 | -0,322529 |
| Signal10 | 0,070295 | 0,52119 | 0,004128 | 0,023006 | 0,098764 |
| Signal11 | -0,33773 | 0,15198 | -0,360046 | -0,220731 | 0,050834 |
| Signal12 | 0,385359 | -0,100694 | -0,28623 | 0,185295 | -0,232151 |

Summierung der Absolutbeträge für alle Signale:

| Signal1 | Signal2 | Signal3 | Signal4 | Signal5 | Signal6 | Signal7 |
|---|---|---|---|---|---|---|
| 1,781179 | 0,770473 | 1,1716956 | 1,2196165 | 1,2060872 | 1,465598 | 0,990382 |

| Signal8 | Signal9 | Signal10 | Signal11 | Signal12 |
|---|---|---|---|---|
| 1,2822101 | 1,451442 | 0,7173837 | 1,1213197 | 1,1897284 |

Sortierung vom größten zum kleinsten Wert - Identifikation der 5 wichtigsten Signale zur späteren Optimierung (hier orange hinterlegt):

| Signal1 | Signal6 | Signal9 | Signal8 | Signal4 | Signal5 | Signal12 |
|---|---|---|---|---|---|---|
| 1,781179 | 1,4655984 | 1,4514416 | 1,2822101 | 1,2196165 | 1,206087 | 1,1897284 |

| Signal3 | Signal11 | Signal7 | Signal2 | Signal10 |
|---|---|---|---|---|
| 1,1716956 | 1,1213197 | 0,9903817 | 0,7704728 | 0,7173837 |

Ermittlung der natürlichen Spannweiten der 5 identifizierten Signale:

| | Signal1 | Signal6 | Signal9 | Signal8 | Signal4 |
|---|---|---|---|---|---|
| Range Minimum | 16411 | 23294 | 7969 | 5834 | 8096 |
| Mittelwert | 16679 | 23781 | 9310 | 7326 | 8733 |
| Range Maximum | 16800 | 24433 | 10361 | 11208 | 9141 |

iv) Bestimmung des Gehalts der Pflanzeninhaltsstoffe, die den Varianzmarkern zugrunde liegen

Dies kann optional durchgeführt werden, so dass den Intensitäten konkrete Gehalte (beispielsweise in mg/L o. ä.) zugeordnet werden. Hierfür stehen entsprechende Quantifizierungsmethoden für diverse analytische Methoden zur Verfügung. Dieser Schritt kann (wie in diesem Beispiel) ggf. aber auch übersprungen werden, da dies nur eine lineare Transformation der Intensitäten darstellt.

v) Festsetzen einer neuen, reduzierten Spannweite

Das erlaubte Minimum wird angehoben, das erlaubte Maximum wird abgesenkt, somit verringert sich die erlaubte Spannweite der Mischung(en) (siehe Abbildung 14):

| | Signal1 | Signal6 | Signal9 | Signal8 | Signal4 |
|---|---|---|---|---|---|
| Neues erlaubtes Minimum | 16545 | 23537 | 8639 | 6580 | 8414 |
| Mittelwert | 16679 | 23781 | 9310 | 7326 | 8733 |
| neues erlaubtes Maximum | 16740 | 24107 | 9835 | 9267 | 8937 |

vi) Durchführung einer Mischungsrechnung

Nach der Berechnung ergibt sich für die zur Mischung heranzuziehende Chargen folgende Handlungsanweisung:

| | Anteil in % |
|---|---|
| Charge 1 | 59,34 |
| Charge 2 | 35,53 |
| Charge 5 | 5,13 |

Erwartetes Ergebnis für dieses Mischungsbeispiel:

| | Signal 1 | Signal 6 | Signal 9 | Signal 8 | Signal 4 |
|---|---|---|---|---|---|
| Erwartete Signalintensitäten | 16740 | 23718 | 8983 | 9040 | 8853 |

Alle erwarteten Signalintensitäten liegen innerhalb der gewünschten reduzierten Spannweite.

vii) Diese Berechnung kann entsprechend wiederholt werden, beispielsweise unter Neu-Mischung anderer Chargen.

**Beispiel C**

**Beispiel für das Vorgehen bei der Varianzreduktion pflanzlicher Rohstoffe durch**

**Mischungsrechnung bei Rumex crispus**

Es werden Ausschnitte der originären Datensätze gezeigt, die das erfindungsgemäße Vorgehen hinreichend vermitteln.

i) Bestimmung von Signalen von Pflanzeninhaltsstoffen mittels LC/MS an mehreren Chargen

Das Ergebnis ist typischerweise eine Tabelle wie die Folgende - die Zahlen sind die gemessenen Intensitätswerte aus beispielsweise einer LC/MS-Messung.

| | Signal 1 | Signal 2 | Signal 3 | Signal 4 | Signal 5 | Signal 6 | Signal 7 | Signal 8 | Signal 9 | Signal 10 | Signal 11 | Signal 12 |
|---|---|---|---|---|---|---|---|---|---|---|---|---|
| Charge_1 | 16711 | 5763 | 14366 | 8912 | 8521 | 24000 | 29252 | 11208 | 9471 | 8302 | 7189 | 5317 |
| Charge_2 | 16797 | 5961 | 14480 | 8713 | 9170 | 23294 | 25059 | 5834 | 7969 | 8250 | 5102 | 3937 |
| Charge_3 | 16411 | 6182 | 14636 | 8096 | 7357 | 23788 | 21161 | 7111 | 9148 | 7458 | 4075 | 5450 |
| Charge_4 | 16800 | 7030 | 12939 | 8800 | 8293 | 24433 | 19279 | 6319 | 9599 | 8005 | 4291 | 6829 |
| Charge_5 | 16677 | 6739 | 13440 | 9141 | 9954 | 23389 | 20415 | 6159 | 10361 | 8324 | 2171 | 6645 |

ii) Aufteilung der Signale in mehrere Teilbereiche und Summierung dieser Teilbereiche

1.1. Für die Aufteilung der Signale in mehrere Teilbereiche stehen verschiedene Strategien zur Verfügung. Beispielsweise könnte man nach Retentionszeit der LC/MS-Signale oder nach Masse sortieren und entsprechend zusammenfassen.

| | Bereich 1 | | | Bereich 2 | | | Bereich 3 | | | Bereich 4 | | |
|---|---|---|---|---|---|---|---|---|---|---|---|---|
| | Signal 1 | Signal 2 | Signal 3 | Signal 4 | Signal 5 | Signal 6 | Signal 7 | Signal 8 | Signal 9 | Signal 10 | Signal 11 | Signal 12 |
| Charge_1 | 16711 | 5763 | 14366 | 8912 | 8521 | 24000 | 29252 | 11208 | 9471 | 8302 | 7189 | 5317 |
| Charge_2 | 16797 | 5961 | 14480 | 8713 | 9170 | 23294 | 25059 | 5834 | 7969 | 8250 | 5102 | 3937 |
| Charge_3 | 16411 | 6182 | 14636 | 8096 | 7357 | 23788 | 21161 | 7111 | 9148 | 7458 | 4075 | 5450 |
| Charge_4 | 16800 | 7030 | 12939 | 8800 | 8293 | 24433 | 19279 | 6319 | 9599 | 8005 | 4291 | 6829 |
| Charge_5 | 16677 | 6739 | 13440 | 9141 | 9954 | 23389 | 20415 | 6159 | 10361 | 8324 | 2171 | 6645 |

1.2. Summierung der Signale aus den Teilbereichen, danach weiter mit Schritt iii)

| | Bereich 1 | Bereich 2 | Bereich 3 | Bereich 4 |
|---|---|---|---|---|
| Charge_1 | 36841 | 41433 | 49932 | 20808 |
| Charge_2 | 37238 | 41177 | 38862 | 17290 |
| Charge_3 | 37229 | 39241 | 37421 | 16983 |
| Charge_4 | 36769 | 41526 | 35197 | 19126 |
| Charge_5 | 36855 | 42485 | 36934 | 17140 |

2.1. Eine weitere Möglichkeit ergibt sich aus der Verwendung des Massendefekt-Plots, mit Hilfe dessen sich die Signale ihrer aufgrund ihrer chemischen Stoffklasse gliedern lässt. Der Massendefekt eines jeden Signals berechnet sich wie folgt:

$$MD = \frac{mz - floor(mz)}{mz} * 10^6$$

*MD* = Massendefekt

*mz* = m/z-Verhältnis auf 4 Nachkommastellen

*floor()* = Funktion, die eine Komma-Zahl auf die nächste Ganzzahl abrundet

Beispielrechnung für einige m/z-Verhältnisse

| *mz* (gemessen) | *floor*(*mz*) | $\frac{mz - floor(mz)}{mz}$ | *MD* (Massendefekt) |
|---|---|---|---|
| 183,1748 | 183 | 0,00095428 | 954,2797372 |
| 187,0981 | 187 | 0,000524324 | 524,3238707 |
| 357,0568 | 357 | 0,000159078 | 159,0783315 |
| 585,1575 | 585 | 0,000269158 | 269,1583035 |
| 839,1849 | 839 | 0,000220333 | 220,3328492 |

Das Ergebnis ist eine Graphik wie in Abbildung 11, bei der jedes Signal in ein Koordinatensystem eingetragen werden kann, bei dem die X-Achse das m/z-Verhältnis und die Y-Achse der zugehörige Massendefekt ist. Die Position des Signals ist dabei i. d. R. charakteristisch für die Stoffgruppe (z.B. Flavonoide, Terpenoide, etc...), zu der das Signal gehört.

2.2. Anschließend kann beispielsweise mit Hilfe des darübergelegten Gitters (hier in grau dargestellt) für jede Zelle die Summe der Intensitäten der darin enthaltenen Signale für jede Charge berechnet werden.
2.3. Übersichtsmäßig kann die Zahl der Signale pro Zelle (pro Zelle können mehrere Signale in unterschiedlicher Intensität enthalten sein) in einer Heatmap dargestellt werden (siehe Abbildung 12).
2.4. Bei der Summierung der Signalintensitäten in den Einzelzellen ergibt sich beispielsweise folgender Ausschnitt (Zellen, deren Summe 0 ist, da sich dort kein Signal befindet wurden exkludiert. Die Beschriftung folgt stets dem Schema "Zelle (Koordinate auf X-Achse | Koordinate auf Y-Achse)")

| | Zelle (1\|5) | Zelle (1\|6) | Zelle (1\|9) | Zelle (2\|8) | Zelle (2\|9) |
|---|---|---|---|---|---|
| Charge 1 | 1430 | 4703 | 541 | 1671 | 4298 |
| Charge 2 | 2577 | 5058 | 950 | 2133 | 4306 |
| Charge 3 | 2664 | 4939 | 1046 | 2502 | 4309 |
| Charge 4 | 2808 | 5186 | 971 | 2526 | 4306 |
| Charge 5 | 1717 | 4316 | 600 | 1817 | 4300 |

Mit dieser Tabelle kann dann analog ab dem folgenden Punkt iii) weitergearbeitet werden, das weitere Vorgehen ist identisch.

iii) Identifizierung der Teilbereiche, die am stärksten zur Varianz / Standardabweichung beitragen

Berechnung der Loadings der Tabelle von Schritt 1.2 (oder 2.4) mittels einer Principal component analysis (hier beispielsweise 4 Hauptkomponenten, mehr Hauptkomponenten können jederzeit gewählt werden):

| | PC1 | PC2 | PC3 | PC4 |
|---|---|---|---|---|
| Bereich 1 | 0,5560277 | -0,3779871 | 0,4745952 | -0,568083 |
| Bereich 2 | -0,4228279 | 0,588995 | 0,6291389 | -0,2801532 |
| Bereich 3 | -0,4177076 | -0,6292769 | 0,4980767 | 0,4259701 |
| Bereich 4 | -0,5810078 | -0,3379663 | -0,3617503 | -0,6460227 |

Summierung der Absolutbeträge für alle Bereiche:

| Bereich 1 | Bereich 2 | Bereich 3 | Bereich 4 |
|---|---|---|---|
| 1,976693 | 1,921115 | 1,9710313 | 1,9267471 |

Sortierung vom größten zum kleinsten Wert - Identifikation der 2 wichtigsten Bereiche zur späteren Optimierung (hier unterstrichen hinterlegt; es können auch mehr Bereiche für die spätere Optimierung gewählt werden):

| Bereich 1 | Bereich 3 | Bereich 4 | Bereich 2 |
|---|---|---|---|
| 1,976693 | 1,9710313 | 1,9267471 | 1,921115 |

iv) Bestimmung des Gehalts der Pflanzeninhaltsstoffe, die den Varianzmarkern zugrunde liegen

Dies kann optional durchgeführt werden, so dass den Intensitäten konkrete Gehalte (beispielsweise in mg/L o. ä.) zugeordnet werden. Hierfür stehen entsprechende Quantifizierungsmethoden für diverse analytische Methoden zur Verfügung. Dieser Schritt kann (wie in diesem Beispiel) ggf. aber auch übersprungen werden, da dies nur eine lineare Transformation der Intensitäten darstellt.

v) Bestimmung der natürlichen Spannweite der gewählten Bereiche

| | Bereich 1 | Bereich 3 |
|---|---|---|
| Range Minimum | 36769 | 35197 |
| Mittelwert | 36986 | 39669 |
| Range Maximum | 37238 | 49932 |

vi) Festlegung einer reduzierten Spannweite

Das erlaubte Minimum wird angehoben, das erlaubte Maximum wird abgesenkt, somit verringert sich die erlaubte Spannweite der Mischung(en) (siehe Abbildung 15):

| | Bereich 1 | Bereich 3 |
|---|---|---|
| Neues erlaubtes Minimum | 36878 | 37433 |
| Mittelwert | 36986 | 39669 |
| neues erlaubtes Maximum | 37112 | 44801 |

vii) Durchführung einer Mischungsrechnung

Nach der Berechnung ergibt sich für die Mischung heranzuziehende Chargen beispielsweise folgende Handlungsanweisung:

| | Anteil in % |
|---|---|
| Charge 1 | 50,00 |
| Charge 2 | 20,00 |
| Charge 5 | 30,00 |

Erwartetes Ergebnis für dieses Mischungsbeispiel:

| | Bereich 1 | Bereich 3 |
|---|---|---|
| Erwartete Signalintensitäten | 36924 | 43819 |

Alle erwarteten Signalintensitäten liegen innerhalb der gewünschten reduzierten Spannweite.

viii) Diese Berechnung kann entsprechend wiederholt werden, beispielsweise unter Neu-Mischung anderer Chargen.

**Beispiel D**

**Beispiele, wie im Rahmen der Mischungsberechnung die LC/MS-Signale erkannt werden, die für die Optimierung herangezogen werden können**

Im Folgenden sind 2 mögliche Beispiele aufgeführt:

a) Durch Auswertung der Principal component analysis (PCA) einer LC/MS-Messung einer Stichprobe von Rohdrogen
b) Durch Bewertung der Standardabweichungen gemessener LC/MS-Signale einer Stichprobe von Rohdrogen

Aus Platzgründen werden hier nur Ausschnitte der originalen Datensätze gezeigt, die das Prinzip vermitteln sollen.

Zu a):

i) Bestimmung von Signalen von Pflanzeninhaltsstoffen mittels LC/MS an mehreren Chargen

Das Ergebnis ist typischerweise eine Tabelle wie die Folgende - die Zahlen sind die gemessenen Intensitätswerte aus beispielsweise einer LC/MS-Messung. (Im folgenden Beispiel wurden 40 Chargen vermessen und jeweils 363 Signale ermittelt, folgende Tabelle ist ein Ausschnitt)

| | Signal 1 | Signal 2 | Signal 3 | Signal 4 | Signal 5 | Signal 6 | Signal 7 | Signal 8 | Signal 9 | Signal 10 | Signal 11 | Signal 12 |
|---|---|---|---|---|---|---|---|---|---|---|---|---|
| Charge_1 | 9418 | 20502 | 15661 | 8338 | 19523 | 5265 | 769 | 4071 | 3142 | 3710 | 53741 | 5275 |
| Charge_2 | 12067 | 24221 | 15079 | 3575 | 19903 | 5549 | 7052 | 3991 | 3373 | 10319 | 55016 | 5898 |
| Charge_3 | 10880 | 18391 | 15361 | 4120 | 19271 | 4581 | 5274 | 3660 | 2731 | 4308 | 52762 | 4763 |
| Charge_4 | 12972 | 19288 | 16197 | 7262 | 21719 | 7748 | 7889 | 4790 | 2897 | 6482 | 57076 | 7193 |
| Charge_5 | 11923 | 17665 | 17322 | 5598 | 21461 | 6120 | 7760 | 4734 | 3110 | 5847 | 56114 | 6207 |

ii) Durchführung einer Principal Component Analysis (PCA)

Darstellung der Scores & Loadings

Insbesondere von den Loadings werden hier 6 Hauptkomponenten berechnet und dargestellt - dies entspricht im vorliegenden Beispiel einer Gesamtvarianz von rund 80% und beschreibt daher den Datensatz hinreichend gut. Das Heranziehen weiterer Hauptkomponenten ist aber jederzeit möglich, um die erklärte Gesamtvarianz zu erhöhen.

| | PC1 | PC2 | PC3 | PC4 | PC5 | PC6 |
|---|---|---|---|---|---|---|
| Anteil Varianz (%) | 27,552 | 23,735 | 10,933 | 7,845 | 5,311 | 4,809 |
| Kumulative Varianz (%) | 27,552 | 51,287 | 62,22 | 70,065 | 75,376 | 80,185 |

Wie im Beispiel A beschrieben wird von den erhaltenen Loading-Werten der Absolutbetrag berechnet, über die gewählten Hauptkomponenten aufsummiert und dann nach Größe absteigend sortiert. In Abbildung 10 wurden die 5 am stärksten zur Varianz beitragenden Signale gewählt und in den Graphiken des Loadingplots (Darstellung von jeweils 2 der 6 Hauptkomponenten pro Plot) rot markiert (die Auswahl weiterer Signale ist jederzeit möglich). Bei den rot markierten Signalen handelt es sich auch in der Tat stets um die gleichen Signale - nur repräsentiert in den unterschiedlichen Hauptkomponenten. Sie decken die komplette Spannweite der Signale ab und repräsentieren die Proben

hinreichend gut.

Die auf diese Weise ausgewählten Signale können dann für die Mischungsberechnung wie im Beispiel A beschrieben verwendet werden.

Zu b):

Eine alternative Signalauswahl ist durch bloße Betrachtung der Standardabweichung der Signalintensitäten aus mittels LC/MS gemessenen Chargen möglich. So können beispielsweise die 5 Signale mit der höchsten Standardabweichung identifiziert werden und ebenfalls für die Mischungsberechnung herangezogen werden.

i) Messung mehrerer Chargen mittels LC/MS (hier: Ausschnitt aus erhaltener Signaltabelle) und Berechnung der Standardabweichungen für jedes Signal

| | Signal 1 | Signal 2 | Signal 3 | Signal 4 | Signal 5 | Signal 6 | Signal 7 | Signal 8 | Signal 9 | Signal 10 | Signal 11 | Signal 12 |
|---|---|---|---|---|---|---|---|---|---|---|---|---|
| Charge_1 | 9418 | 20502 | 15661 | 8338 | 19523 | 5265 | 769 | 4071 | 3142 | 3710 | 53741 | 5275 |
| Charge_2 | 12067 | 24221 | 15079 | 3575 | 19903 | 5549 | 7052 | 3991 | 3373 | 10319 | 55016 | 5898 |
| Charge_3 | 10880 | 18391 | 15361 | 4120 | 19271 | 4581 | 5274 | 3660 | 2731 | 4308 | 52762 | 4763 |
| Charge_4 | 12972 | 19288 | 16197 | 7262 | 21719 | 7748 | 7889 | 4790 | 2897 | 6482 | 57076 | 7193 |
| Charge_5 | 11923 | 17665 | 17322 | 5598 | 21461 | 6120 | 7760 | 4734 | 3110 | 5847 | 56114 | 6207 |
| Standard-abweichung | 1358 | 2580 | 884 | 2024 | 1135 | 1196 | 2973 | 493 | 246 | 2595 | 1741 | 928 |

ii) Sortierung / Identifizierung der Signale mit der höchsten Standardabweichung

In diesem Beispiel wurden 5 Signale gewählt (mehr sind jederzeit möglich) und unterstrichen markiert.

| | Standardabweichung |
|---|---|
| Signal 7 | 2972,69048 |
| Signal 10 | 2594,5976 |
| Signal 2 | 2579,53471 |
| Signal 4 | 2024,25463 |
| Signal 11 | 1741,25076 |
| Signal 1 | 1357,8297 |
| Signal 6 | 1195,66479 |
| Signal 5 | 1135,03692 |
| Signal 12 | 927,916591 |
| Signal 3 | 884,462549 |
| Signal 8 | 493,222769 |
| Signal 9 | 245,744379 |

Mit diesen gewählten Signalen kann ebenfalls eine Mischungsberechnung durchgeführt werden, wie an anderer Stelle bereits dargelegt. Diese zweite Methode identifiziert weitgehend die gleichen Signale wie das Verfahren über die Principal component analysis (PCA).

Werden beispielsweise 20 statt nur 5 Signale über die beiden Verfahren ausgewählt, so zeigt sich bei der Auswahl in diesem Beispiel dennoch eine Übereinstimmung von 17 Signalen (siehe Abbildung 9).

i) Messung mehrerer Chargen mittels LC/MS (hier: Ausschnitt aus erhaltener Signaltabelle) und Berechnung der Standardabweichungen für jedes Signal

| | Signal 1 | Signal 2 | Signal 3 | Signal 4 | Signal 5 | Signal 6 | Signal 7 | Signal 8 | Signal 9 | Signal 10 | Signal 11 | Signal 1 |
|---|---|---|---|---|---|---|---|---|---|---|---|---|
| Charge_1 | 9418 | 20502 | 15661 | 8338 | 19523 | 5265 | 769 | 4071 | 3142 | 3710 | 53741 | 5275 |
| Charge_2 | 12067 | 24221 | 15079 | 3575 | 19903 | 5549 | 7052 | 3991 | 3373 | 10319 | 55016 | 5898 |
| Charge_3 | 10880 | 18391 | 15361 | 4120 | 19271 | 4581 | 5274 | 3660 | 2731 | 4308 | 52762 | 4763 |
| Charge_4 | 12972 | 19288 | 16197 | 7262 | 21719 | 7748 | 7889 | 4790 | 2897 | 6482 | 57076 | 7193 |
| Charge_5 | 11923 | 17665 | 17322 | 5598 | 21461 | 6120 | 7760 | 4734 | 3110 | 5847 | 56114 | 6207 |
| Standard-abweichung | 1358 | 2580 | 884 | 2024 | 1135 | 1196 | 2973 | 493 | 246 | 2595 | 1741 | 928 |

ii) Sortierung / Identifizierung der Signale mit der höchsten Standardabweichung

In diesem Beispiel wurden 5 Signale gewählt (mehr sind jederzeit möglich) und unterstrichen markiert.

| | Standardabweichung |
|---|---|
| Signal 7 | 2972,69048 |
| Signal 10 | 2594,5976 |
| Signal 2 | 2579,53471 |
| Signal 4 | 2024,25463 |
| Signal 11 | 1741,25076 |
| Signal 1 | 1357,8297 |
| Signal 6 | 1195,66479 |
| Signal 5 | 1135,03692 |
| Signal 12 | 927,916591 |
| Signal 3 | 884,462549 |
| Signal 8 | 493,222769 |
| Signal 9 | 245,744379 |

Mit diesen gewählten Signalen kann ebenfalls eine Mischungsberechnung durchgeführt werden, wie an anderer Stelle bereits dargelegt. Diese zweite Methode identifiziert weitgehend die gleichen Signale wie das Verfahren über die Principal component analysis (PCA).

Werden beispielsweise 20 statt nur 5 Signale über die beiden Verfahren ausgewählt, so zeigt sich bei der Auswahl in diesem Beispiel dennoch eine Übereinstimmung von 17 Signalen (siehe Abbildung 9).

## Patentansprüche

1. Verfahren zur Herstellung von Pflanzenmaterial mit reduzierter Varianz von Gehalten an Pflanzeninhaltsstoffen, **dadurch gekennzeichnet, dass** mindestens ein Varianzmarker verwendet wird und ein Mischen von mindestens zwei Chargen mittels eines Mischungsrechners erfolgt.

2. Verfahren zur Herstellung von Pflanzenmaterial mit reduzierter Varianz von Gehalten an Pflanzeninhaltsstoffen mit den folgenden Schritten:

   i.) Bestimmung von Signalintensitäten zu Pflanzeninhaltsstoffen in zwei oder mehr Chargen mittels eines Detektors, insbesondere GC-MS und / oder LC-MS,
   ii.) Identifizierung von mindestens einem Pflanzeninhaltsstoff, der einen Beitrag, vorzugsweise den größten Beitrag zur Varianz leistet und dessen Bestimmung der natürlichen Spannweite,
   iii.) Festsetzen eines oder mehrerer Grenzwerte, welche kleiner als die jeweilige natürliche Spannweite aus ii.) ist,
   iv.) Mischen von mindestens zwei Chargen, wobei mittels eines Mischungsrechners mindestens ein Grenzwert aus iii.) berücksichtigt wird, v.) Optional, Wiederholung der Schritte i.) bis iv.).

3. Verfahren zur Herstellung von Pflanzenmaterial mit reduzierter Varianz von Gehalten an Pflanzeninhaltsstoffen mit den folgenden Schritten:

   i.) Bestimmung von Signalintensitäten zu Pflanzeninhaltsstoffen in zwei oder mehr Chargen mittels eines Detektors, insbesondere GC-MS und / oder LC-MS,
   ii.) Aufteilung der Signale in zwei oder mehrere Teilbereiche, sowie Aufsummierung der Signalintensitäten der Pflanzeninhaltsstoffe innerhalb eines jeden Teilbereichs,

iii.) Identifizierung von mindestens einem Teilbereich, der einen Beitrag, vorzugsweise den größten Beitrag zur Varianz leistet, und dessen Bestimmung der natürlichen Spannweite
iv.) Festsetzen eines oder mehrerer Grenzwerte, welche kleiner als die jeweilige natürliche Spannweite aus iii.) ist,
v.) Mischen von mindestens zwei Chargen, wobei mittels eines Mischungsrechners mindestens ein Grenzwert aus iv.) berücksichtigt wird, vi.) Optional, Wiederholung der Schritte i.) bis v.).

4. Verfahren zur Herstellung von Pflanzenmaterial mit reduzierter Varianz von Gehalten an Pflanzeninhaltsstoffen, **dadurch gekennzeichnet, dass** mindestens ein Detektor ausgewählt ist aus der Gruppe Flüssigchromatographie (LC), insbesondere HPLC, vorzugsweise in Verbindung mit hochauflösender Massenspektrometrie, wie Time of Flight (TOF) Geräten, insbesondere die hochauflösende HPLC-TOF-MS, UV-VIS, Wärmeleitfähigkeitsdetektor, Flammenionisationsdetektor, oder solche Detektoren die Signalintensitäten in Abhängigkeit der Retentionszeit in einem Chromatogramm darstellen.

5. Verfahren zur Herstellung von Pflanzenmaterial mit reduzierter Varianz von Gehalten an Pflanzeninhaltsstoffen nach einem der vorstehenden Ansprüche, **dadurch gekennzeichnet, dass** in einer Charge mindestens 100, 200 oder 300 Signalintensitäten oder mehr bestimmt werden.

6. Verfahren zur Herstellung von Pflanzenmaterial mit reduzierter Varianz von Gehalten an Pflanzeninhaltsstoffen nach einem der vorstehenden Ansprüche, **dadurch gekennzeichnet, dass** die Signalintensitäten in 5 oder 10 Chargen bestimmt werden.

7. Verfahren zur Herstellung von Pflanzenmaterial mit reduzierter Varianz von Gehalten an Pflanzeninhaltsstoffen nach einem der vorstehenden Ansprüche, **dadurch gekennzeichnet, dass** das Pflanzenmaterial ausgewählt ist aus der Gruppe Equiseti, Juglandis, Millefolii, Quercus, Taraxaci, Althaeae, Matricariae, Centaurium, Levisticum, Rosmarinus, Angelica, Artemisia, Astragalus, Leonurus, Salvia, Saposhnikovia, Scutellaria, Siegesbeckia, Armoracia, Capsicum, Cistus, Echinacea, Galphimia, Hedera, Melia, Olea, Pelargonium, Phytolacca, Primula, Salix, Thymus, Vitex, Vitis, Rumicis, Verbena, Sambucus, Gentiana, Cannabis, Silybum.

8. Verfahren zur Herstellung von Pflanzenmaterial mit reduzierter Varianz von Gehalten an Pflanzeninhaltsstoffen nach einem der vorstehenden Ansprüche, **dadurch gekennzeichnet, dass** das Pflanzenmaterial ein Pflanzenextrakt ist.

9. Verfahren zur Herstellung von Pflanzenmaterial mit reduzierter Varianz von Gehalten an Pflanzeninhaltsstoffen nach einem der vorstehenden Ansprüche, **dadurch gekennzeichnet, dass** eine Hauptkomponentenanalyse (Principal component analysis (PCA)) verwendet wird.

10. Pflanzenmaterial mit reduzierter Varianz von Gehalten an Pflanzeninhaltsstoffen, erhältlich durch ein Verfahren nach einem der Ansprüche 1-9.

11. Pflanzenmaterial mit reduzierter Varianz von Gehalten an Pflanzeninhaltsstoffen oder mit reduzierter Chargenvariabilität.

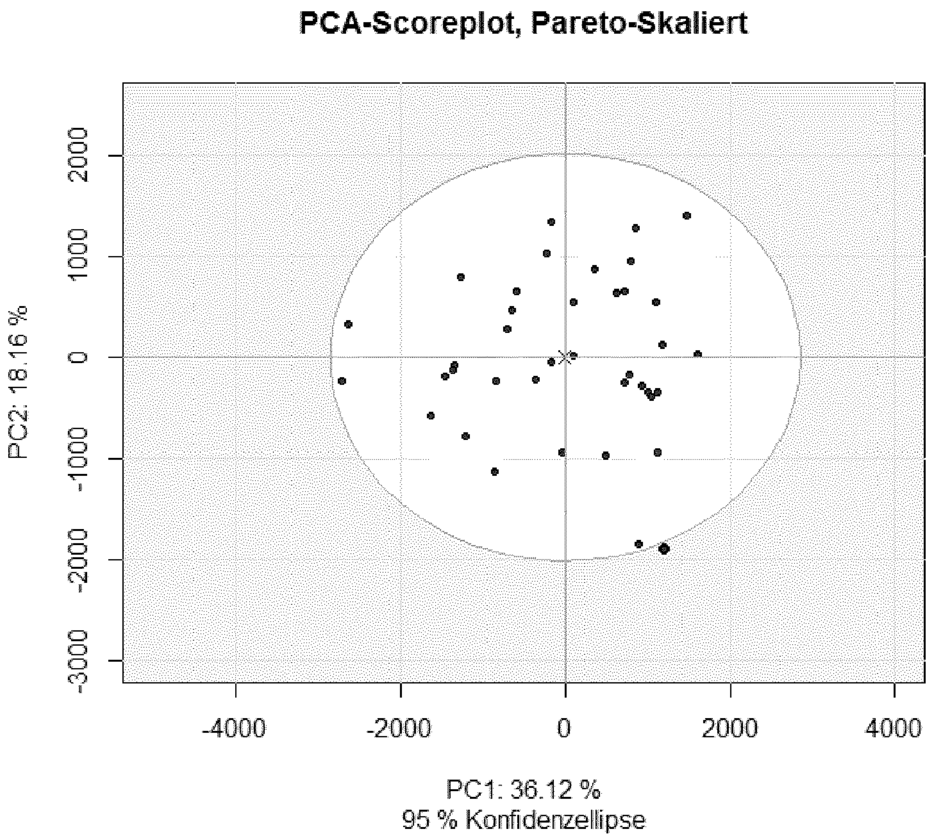


**Abbildung 1: Beispiel Score-Plot einer PCA einer ungemischten Droge**

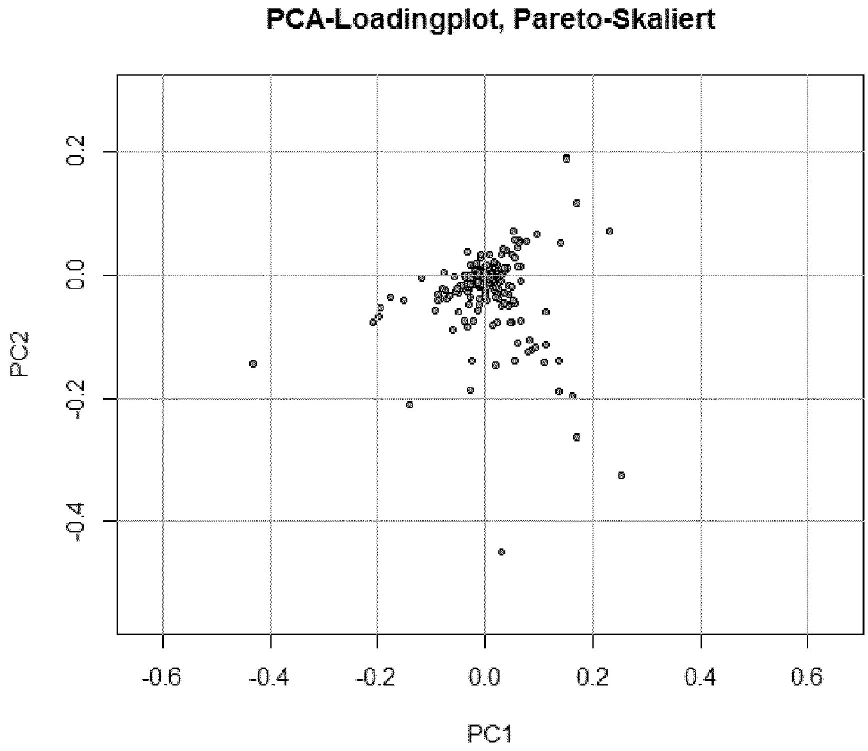


**Abbildung 2: Beispiel Loading-Plot einer PCA einer ungemischten Droge**

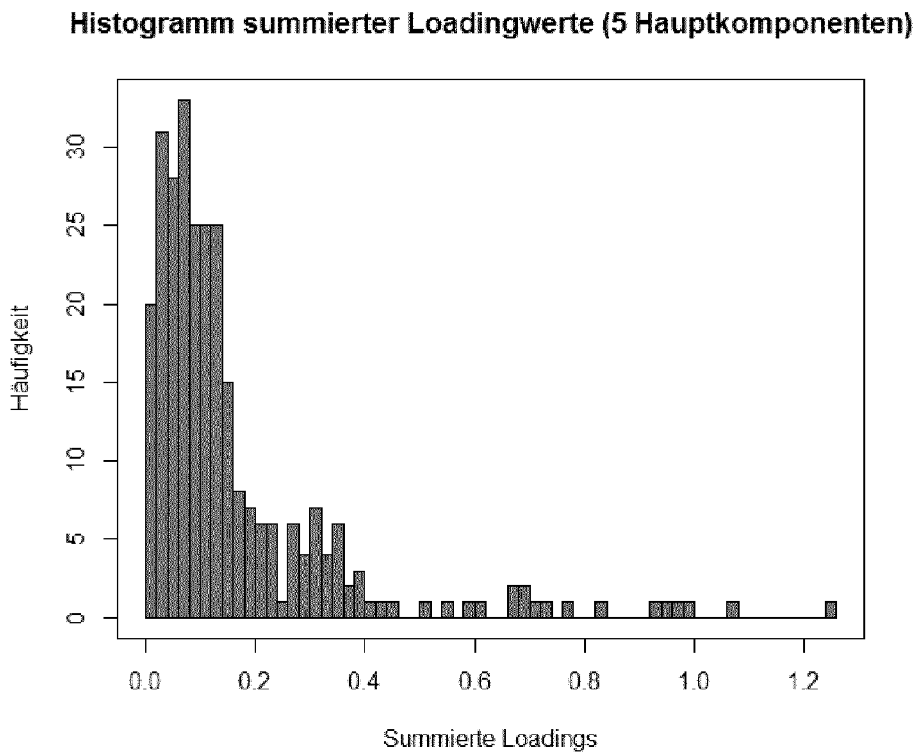


**Abbildung 3: Beispiel-Histogramm summierter Loadingwerte. Als Varianzmarker kommen Signale ganz rechts im Diagramm in Betracht.**

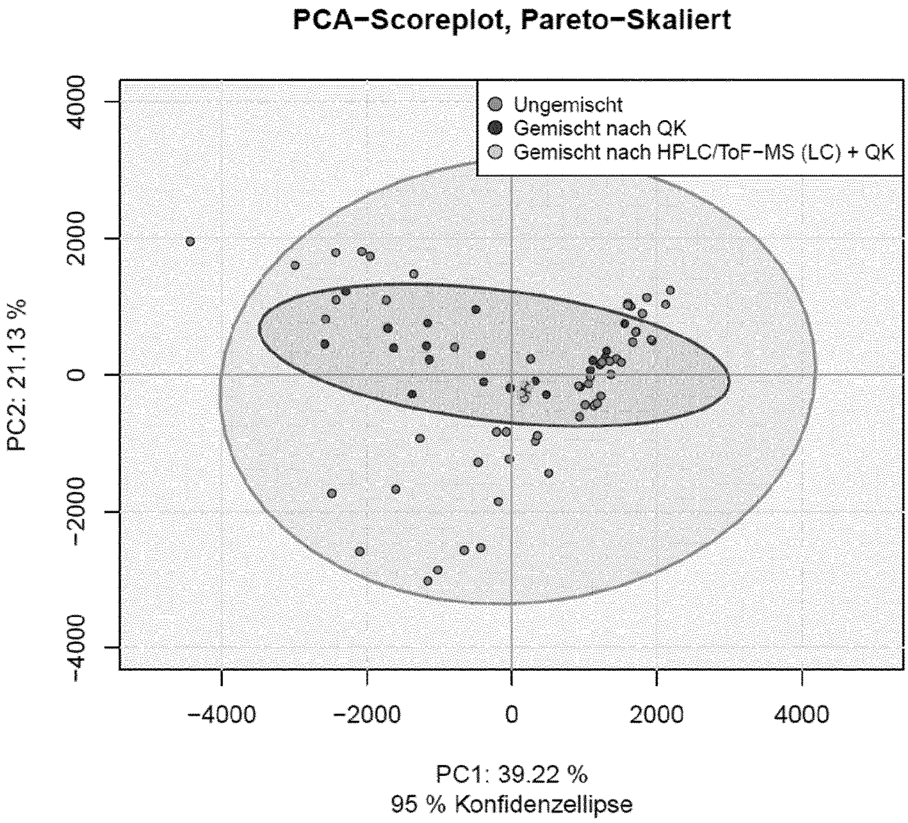


**Abbildung 4: Beispiel Score-Plot einer PCA der Mischungen von Primula veris**

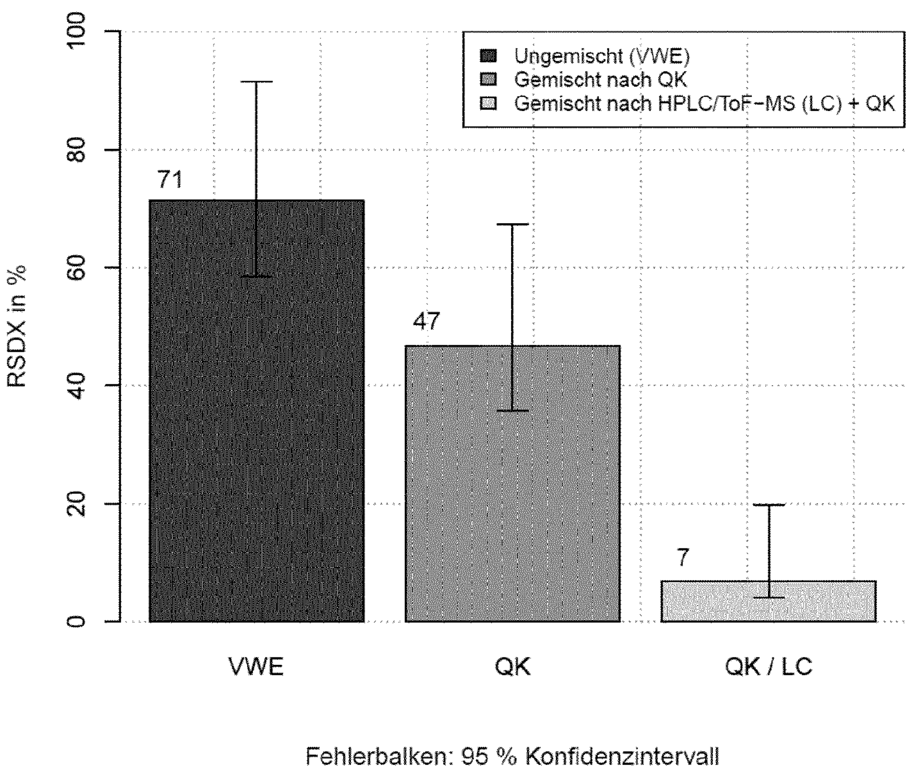


**Abbildung 5: Mischungserfolg Primula veris**

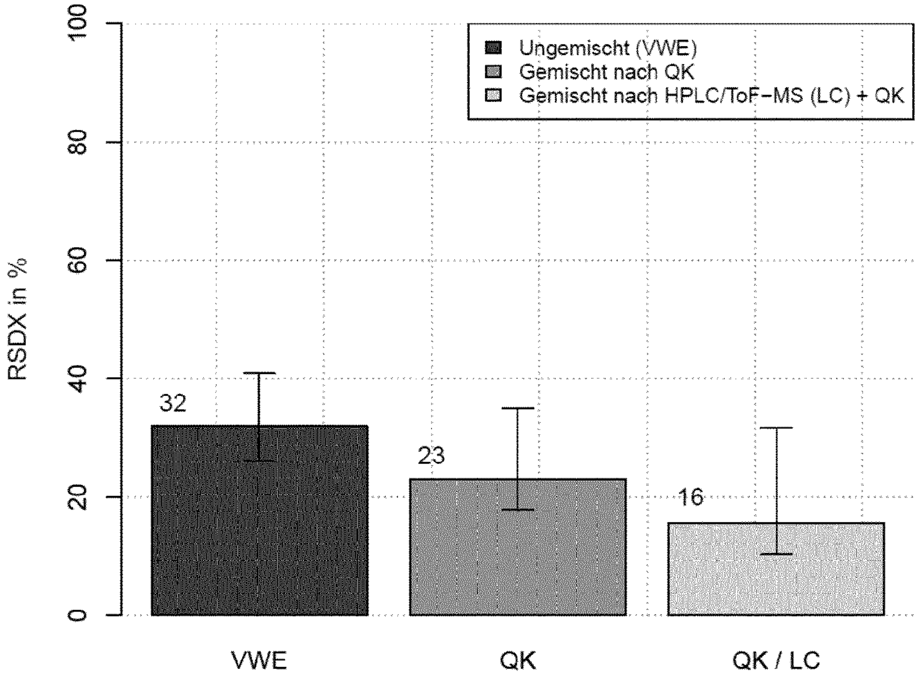


Fehlerbalken: 95 % Konfidenzintervall

**Abbildung 6: Mischungserfolg Rumex crispus**

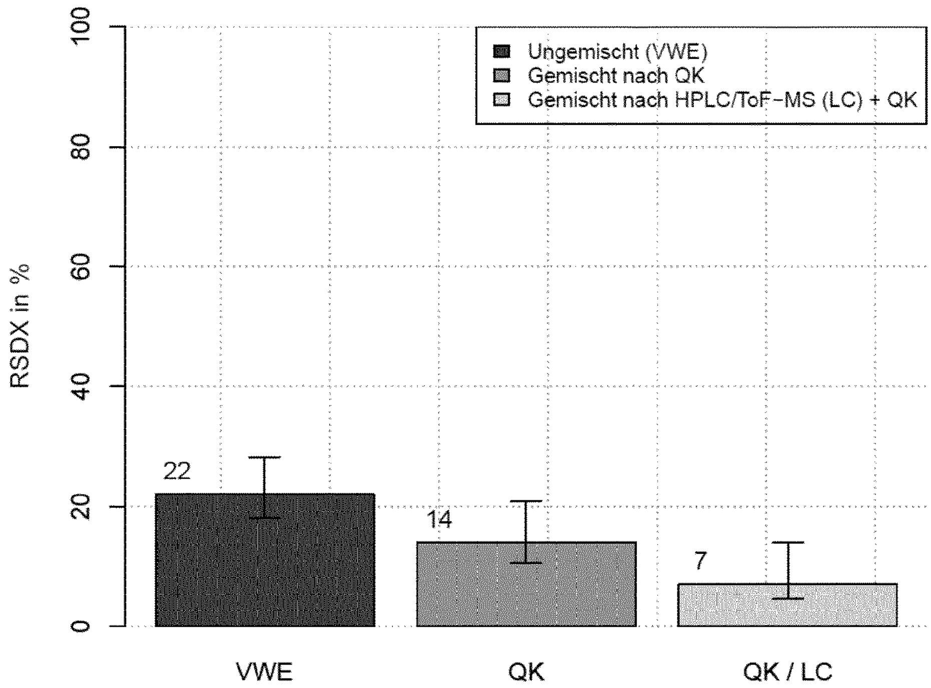


Fehlerbalken: 95 % Konfidenzintervall

**Abbildung 7: Mischungserfolg Sambuccus nigra**

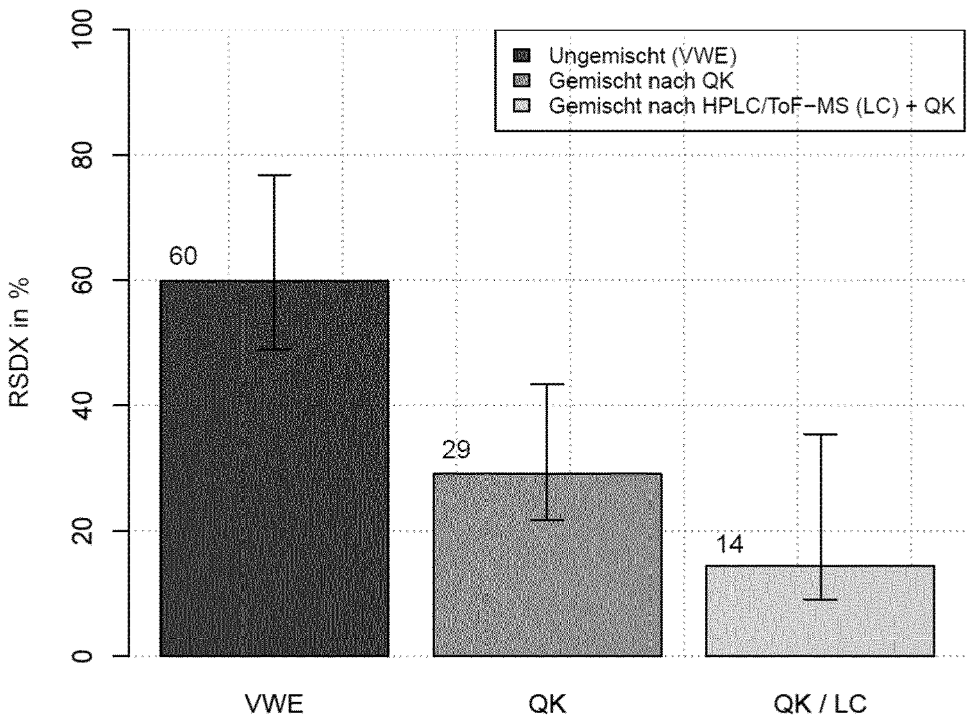


Fehlerbalken: 95 % Konfidenzintervall

**Abbildung 8: Mischungserfolg Verbena officinalis**

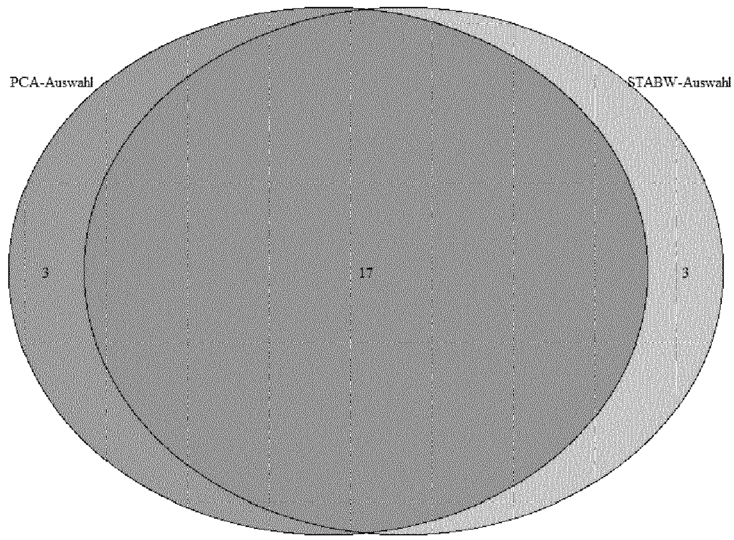


**Abbildung 9: Venn-Diagramm Signalauswahl**

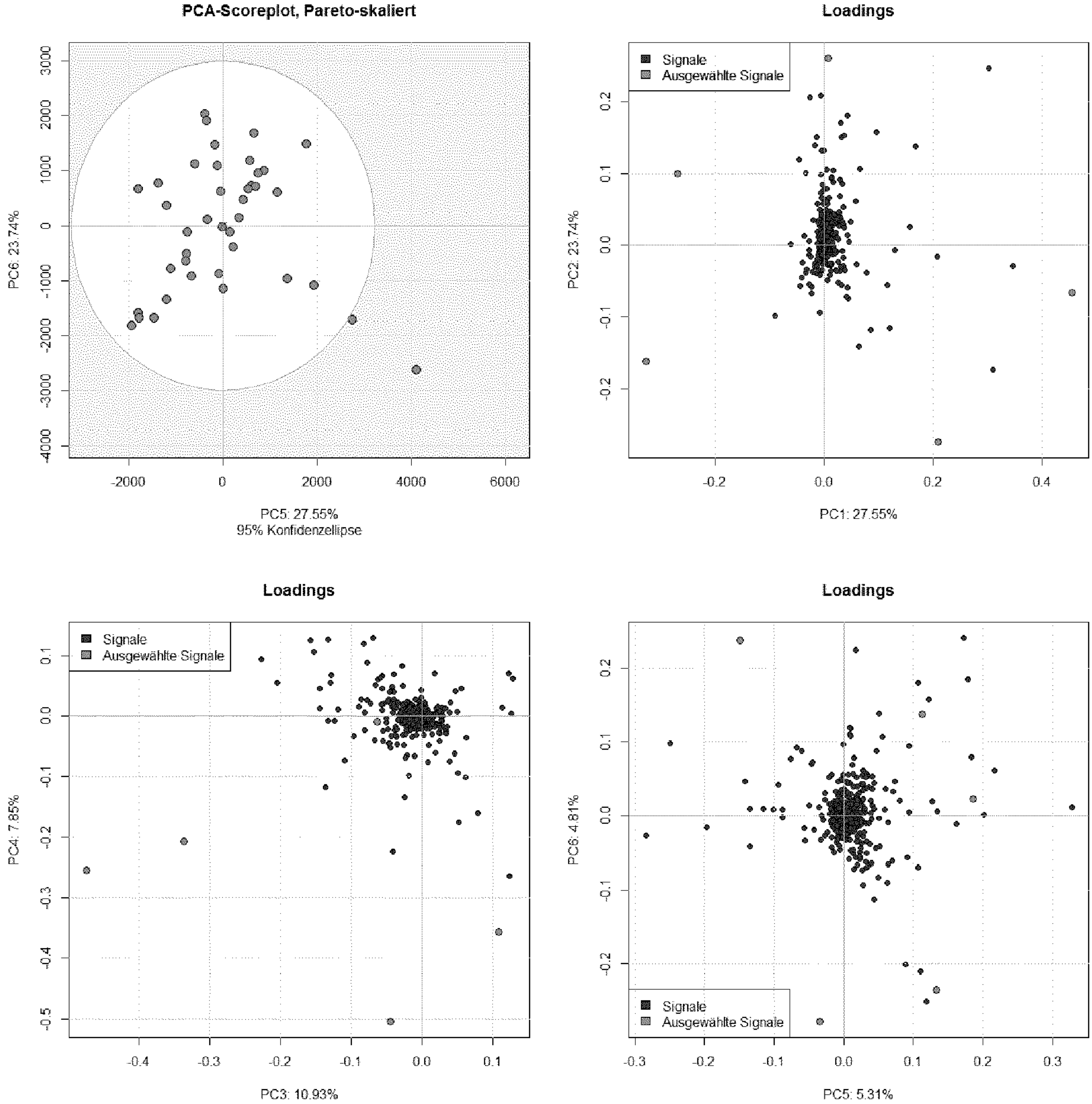


**Abbildung 10: PCA-Plots mit ausgewählten Signalen**

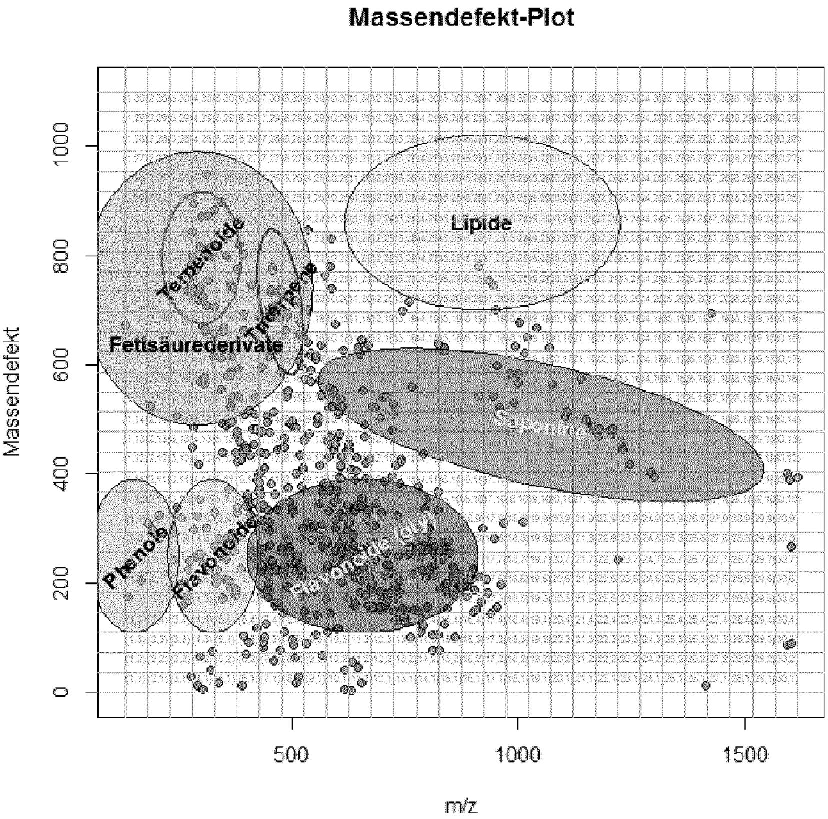


**Abbildung 11: Massendefektplot**

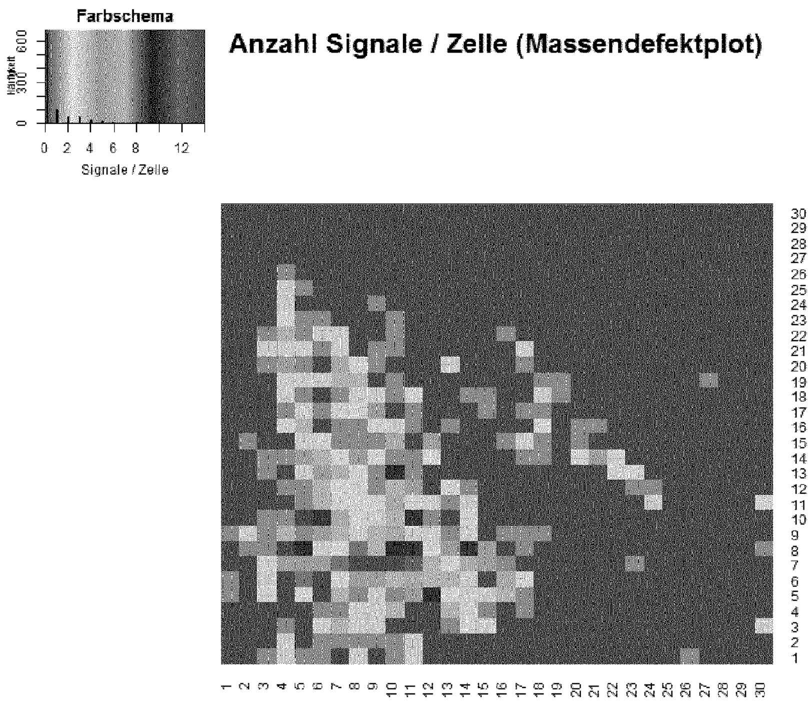


**Abbildung 12: Anzahl Signale / Zelle im Massendefektplot**

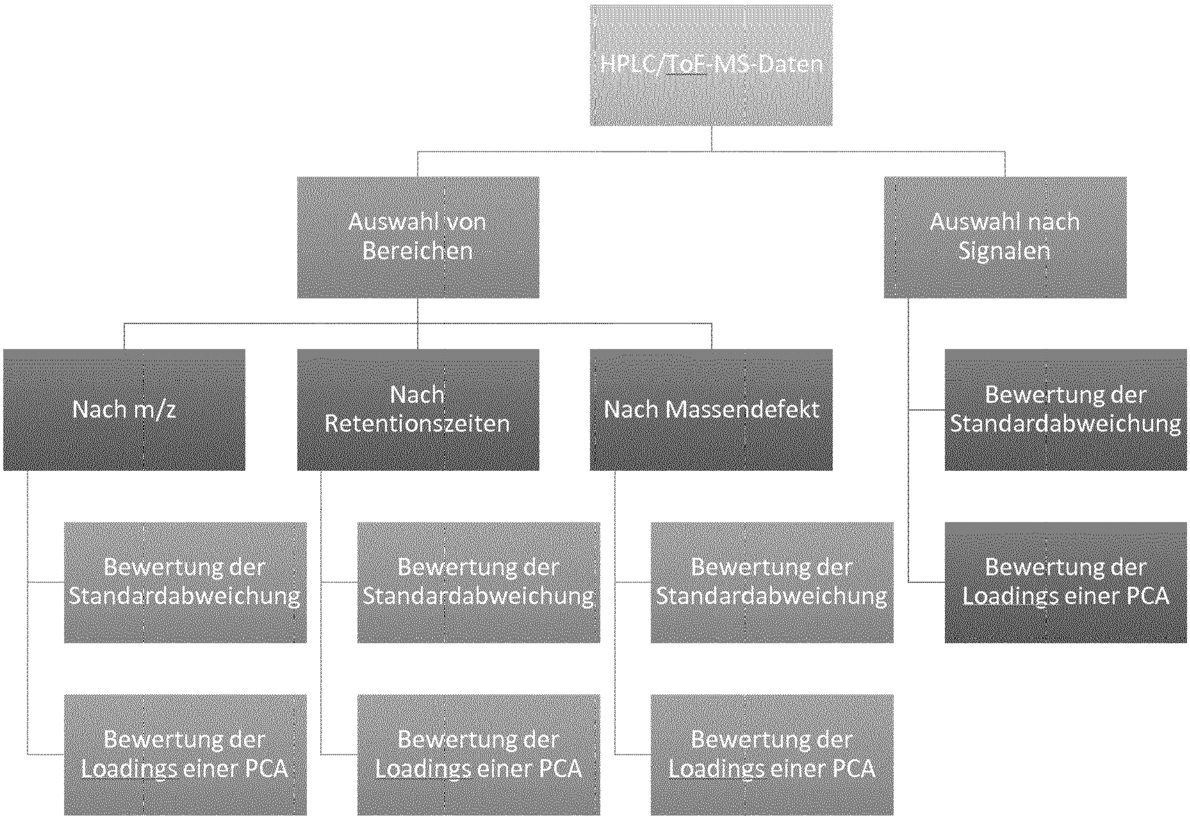


**Abbildung 13: Beispielhafte Übersicht Auswahl von Varianzmarkern**

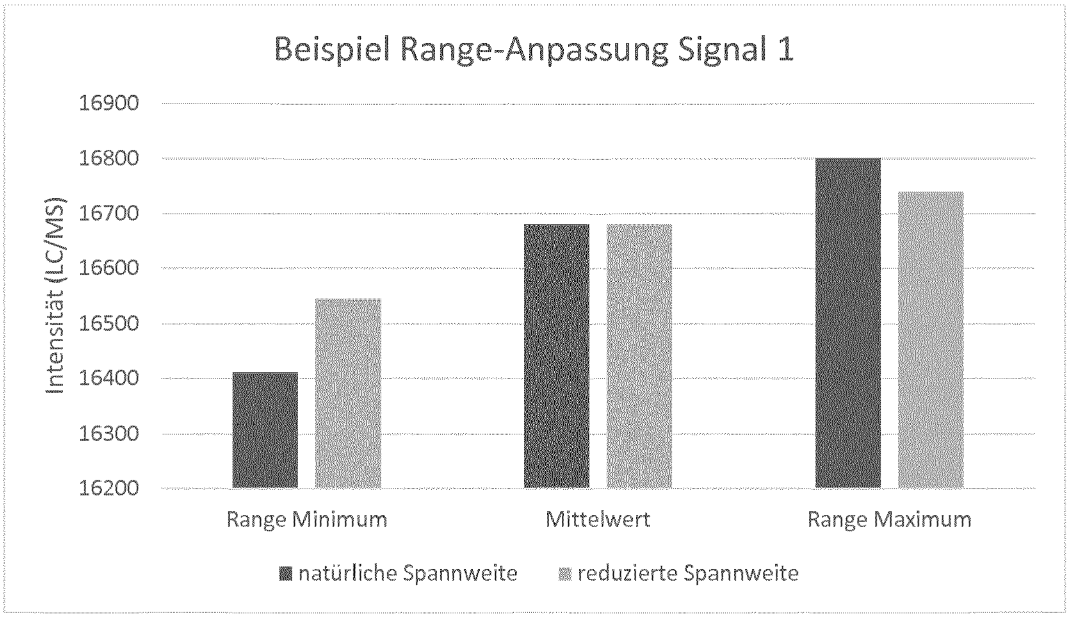


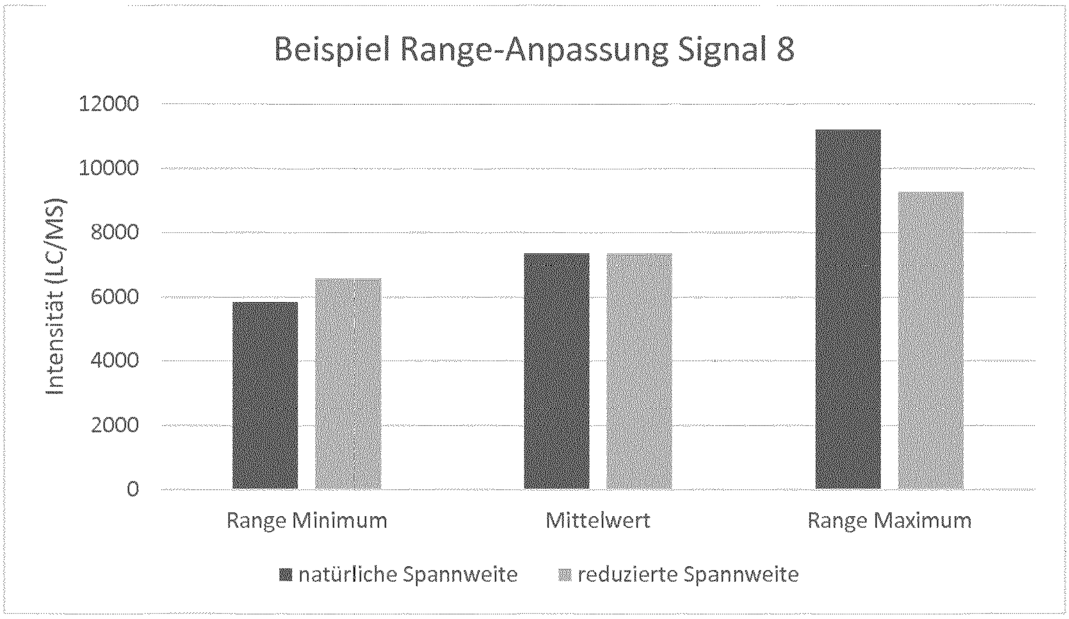


**Abbildung 14 zu Beispiel B v**

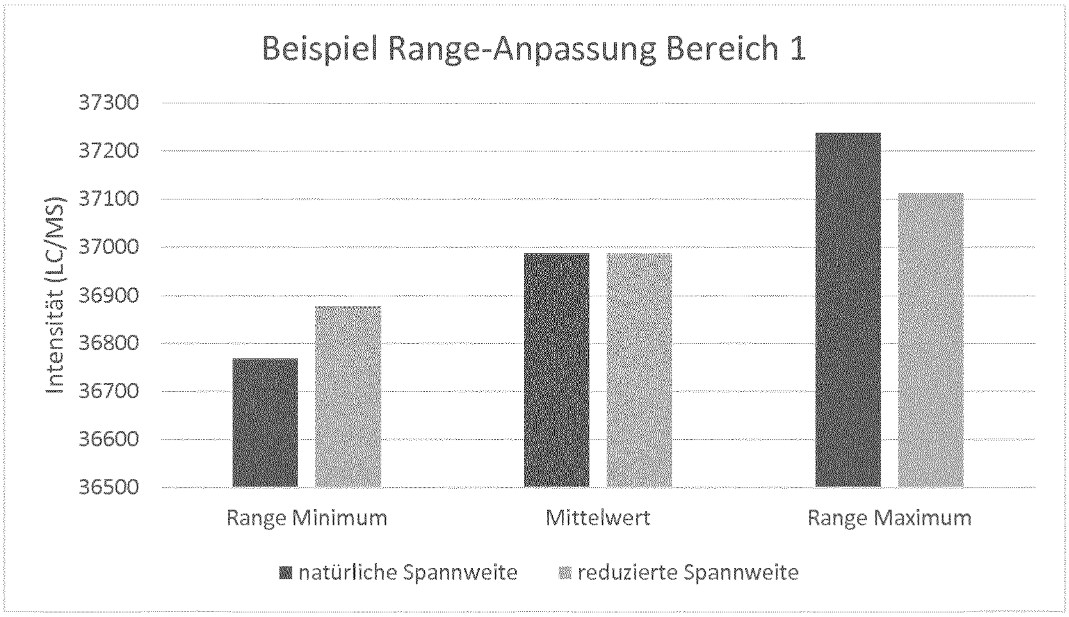


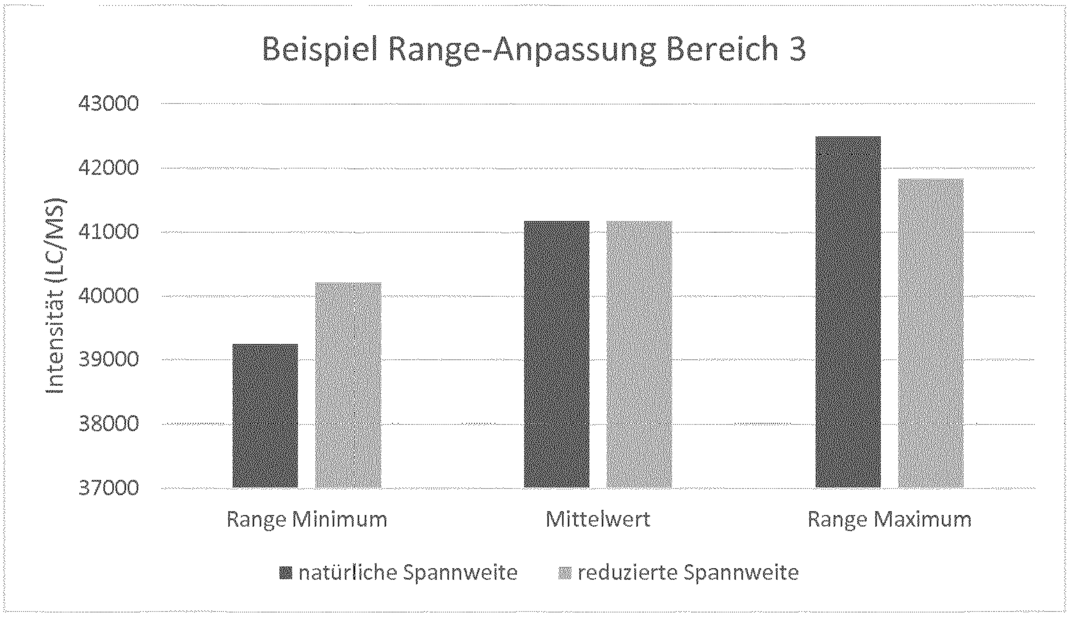


**Abbildung 15 zu Beispiel C vi**

Europäisches Patentamt
European Patent Office
Office européen des brevets

# EUROPÄISCHER RECHERCHENBERICHT

Nummer der Anmeldung
EP 19 15 4942

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | Frank Waimer: "Manufacturing of Herbal Extracts: Types, Techniques and Compliance", , 29. März 2011 (2011-03-29), XP055611568, Gefunden im Internet: URL:https://www.soci.org/-/media/Files/Conference-Downloads/2011/Medicinal-Plants-Mar-11/Frank_Waimer.ashx?la=en [gefunden am 2019-08-07] * Seite 19 - Seite 22 * | 1-3,5-11 | INV. A61K36/18 B01F13/00 |
| X | Fraucke Gaedcke: "Defintionen und Probleme mit den Europäischen Arzneibuch-Monographien für pflanzliche Extrakte und ihre Konsequenzen für die Zulassung", , 4. Oktober 2006 (2006-10-04), XP055611465, Gefunden im Internet: URL:https://www.tu-braunschweig.de/Medien-DB/pharmchem/dphg_2006_gaedcke.pdf [gefunden am 2019-08-07] | 1-3,5-11 | |
| Y | * Seite 5 * * Seite 14 - Seite 20 * | 1-11 | RECHERCHIERTE SACHGEBIETE (IPC) A61K B01F |
| X | Anonymous: "Pflanzliche Arzneimittel: Neue Extrakt-Definition sorgt für Verwirrung", Deutsche Apotheker Zeitung, 21. Juli 2002 (2002-07-21), Seite 26, XP055611351, Gefunden im Internet: URL:https://www.deutsche-apotheker-zeitung.de/daz-az/2002/daz-30-2002/uid-8070 [gefunden am 2019-08-07] | 1,10,11 | |
| Y | * das ganze Dokument * | 1-11 | |

-/--

Der vorliegende Recherchenbericht wurde für alle Patentansprüche erstellt

1

| Recherchenort | Abschlußdatum der Recherche | Prüfer |
|---|---|---|
| München | 7. August 2019 | Winger, Rudolf |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 19 15 4942

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X<br>Y | WO 00/35467 A1 (CELESTIAL SEASONINGS INC [US]) 22. Juni 2000 (2000-06-22)<br>* Seite 24, Zeile 13 - Zeile 20; Ansprüche; Beispiele; Tabelle 1 *<br>----- | 1-11<br>1-11 | |
| | | | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 7. August 2019 | Winger, Rudolf |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR.

EP 19 15 4942

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-08-2019

| Im Recherchenbericht angeführtes Patentdokument | Datum der Veröffentlichung | Mitglied(er) der Patentfamilie | Datum der Veröffentlichung |
|---|---|---|---|
| WO 0035467 A1 | 22-06-2000 | AU 2355900 A | 03-07-2000 |
| | | WO 0035467 A1 | 22-06-2000 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0753306 B1


### In der Beschreibung aufgeführte Nicht-Patentliteratur


- **LAY ; DAVID C.** Linear Algebra and Its Applications. Addison Wesley, 22. August 2005